# EUROPEAN PATENT SPECIFICATION

(11) **EP 4 370 189 B1**
(45) Date of publication and mention of the grant of the patent: **06.05.2026**
(21) Application number: 22850120.1
(22) Date of filing: 25.07.2022
(51) Int. Cl.: A61M 25/06, A61M 25/00

(54) **SAFETY CATHETER ASSEMBLY WITH PASSIVE BLOOD CONTROL**
SICHERHEITSKATHETERANORDNUNG MIT PASSIVER BLUTKONTROLLE
ENSEMBLE CATHÉTER DE SÉCURITÉ AVEC RÉGULATION PASSIVE DU SANG

(30) Priority: 30.07.2021 US 202163227497 P
(43) Date of publication of application: 22.05.2024
(73) Proprietor: Smiths Medical ASD, Inc., Plymouth, MN 55442 (US)
(72) Inventor: ROEHL, Christopher, D., Plymouth, MN 55442 (US); KOEHLER, Thomas, T., Plymouth, MN 55442 (US)
(74) Representative: Venner Shipley LLP
(86) International application number: PCT/US2022/038117
(87) International publication number: WO 2023/009402

(56) References cited:
- US-A1- 2013 079 720
- US-A1- 2016 354 539
- US-A1- 2016 354 539
- US-A1- 2019 160 264
- US-A1- 2020 230 367
- US-A1- 2021 100 985
- US-A1- 2021 100 985

## Description

### Field of the Invention

The present invention relates to safety catheter assemblies and catheter devices including for example peripheral intravenous catheters (PIVC's) and, more particularly, to a safety catheter assembly having a guarded passive release mechanism that shields the sharp distal tip of the needle of the device from at least when the device is ready to be used to the disposal of the device after use.

### Background of the Invention

Conventional PIVC's devices include a catheter assembly, typically having a catheter hub and a catheter tube extending distally thereof, and a needle assembly mounted together in an over-the-needle fashion. The needle assembly typically includes a needle hub or support and a needle cannula extending distally from the needle hub. In a ready to use position, a PIVC has the needle cannula extending through the catheter tube to expose its sharp distal tip distally beyond the distal end of the catheter tube. The sharp distal tip of the needle cannula is used to penetrate the tissue of the patient for insertion of the catheter tube within the vasculature system of a patient. Once the catheter tube is disposed within the vasculature, the needle cannula is withdrawn from the catheter assembly while the catheter assembly remains in fluid communication with the vasculature. The PIVC may also include a needle protector to enclose at least the sharp distal tip of the needle cannula, if not the entire cannula, after use. A PIVC with a protector may be referred to as a safety catheter device or simply a safety catheter.

The catheter hub typically has an open proximal end adapted to receive a male luer taper into the interior cavity of the catheter hub to establish a fluid connection between the patient's vasculature and the luer taper. The proximal end may also be provided with external ears, flange or the like to secure the luer taper in the catheter hub, for example when the luer taper is coupled with a male luer lock collar or nut to form part of a male luer lock connector of an administration set or the end of a syringe, or the like. Under normal conditions, after withdrawal of the needle cannula and before a luer taper is inserted into the catheter hub, blood immediately starts flowing through the catheter tube and into the interior cavity of the catheter hub.

As described in US10,080,867, assigned to the same assignee as the present application, a seal member is disposed in and non-removably held to the interior cavity of the catheter hub. The seal member includes a membrane and at least a distal portion extending distally from the membrane to form a sealed cavity between the membrane and the distal end of the catheter hub. An actuator having a substantially tubular configuration has its distal end secured to the distal end of the catheter hub where the proximal end of the catheter is also secured. The proximal opened end of the actuator is positioned distal of the membrane, so that were the proximal end of the seal member impacted or driven by a force in the distal direction, the membrane would be biased against the proximal opened end of the actuator to an opened position to effect a through passage between the distal end of the catheter and the interior cavity of the catheter hub. Once the distal directional force is removed from the proximal end of the seal member, the distal portion of the seal member would return to its natural position so as to move the membrane away from the opened proximal end of the actuator. No longer biased by the actuator, the membrane reseals to form a partition to prevent back flow of fluid into the interior cavity of the catheter hub proximally beyond the membrane.

In some cases, the safety catheter device may include a needle tip protector through which at least a portion of the needle cannula, or simply needle, passes. The tip protector is configured to enclose or shield the sharp distal tip of the needle after its withdrawal from the catheter tube. The proximal end of the needle is attached to a needle hub, which may be a standalone hub or one that is integrated to a needle housing. The combination of the needle, the needle tip protector and the needle hub may be referred to as the needle assembly or needle insertion assembly or device. Conventionally, once the sharp distal tip of the needle is enclosed in the needle tip protector, the needle tip protector is withdrawn from the catheter hub. Thus, if the needle hub is a standalone hub, the needle tip protector and the portion of the needle between the needle hub and the tip protector is exposed. Even when there is a needle housing, the tip protector is exposed between the time it is withdrawn from the catheter hub and when it is pulled into the distal end of the needle housing. Fluid such as for example contaminated blood that may have adhered to the distal portion of the needle conceivably may be exposed to the environment.

Other prior art arrangements are disclosed in US 2021/100985 and US 2016/354539.

### Summary of the Present Invention

According to the present invention there is provided a safety catheter device according to claim 1.

The safety catheter device of the present invention may be a PIVC that has a catheter hub having an interior cavity defined between its distal and proximal end. The catheter extends from the distal end of the catheter hub so that a through fluid passage may be established between the distal end of the catheter and the interior cavity of the catheter hub. A substantially cylindrical seal member having a body defining a bore is fittingly attached to the inner wall of the interior cavity of the catheter hub. The proximal portion of the seal member opens to the opened proximal end of the catheter hub while the membrane closes off the distal portion of the seal member and the portion of the interior cavity of the catheter hub distal of the membrane.

A needle feature or transition is provided proximate the distal tip of the needle. The needle feature has a dimensional configuration, for example a bump, that prevents the needle feature to pass through the opening at the back wall of the needle tip protector when the needle is moved proximally relative to the catheter hub. Once the needle feature is caught at the opening, further proximal movement of the needle would cause the needle tip protector to move proximally relative to the catheter hub. Before the needle feature reaches the back wall of the needle tip protector, the distal tip of the needle would pass by the barriers of the needle tip protector which act to bias the bosses outwardly to engage with the seal member. As a result, the bosses return to their natural position away from the inner wall of the seal member, thereby freeing the needle tip protector from the seal member and therefore the catheter hub. The needle tip protector, with the sharp distal tip of the needle enclosed therein, may then be removed from the catheter hub.

The guard may be configured to have a chamber at its proximal end adapted to accept at least the proximal portion of the needle tip protector. At least a pair of opposing side wing extensions, covers or shields extend distally from the chamber to overlap the proximal end portion of the catheter hub when the catheter assembly is in its ready to use position. A push tab may be provided between the opposing covers to enable a user to press her thumb or one of her fingers to move the device in a distal direction to guide the distal tip of the needle, and the distal portion of the catheter through which the needle slidably extends, into the vein or artery of the patient.

A needle tip protector cup, or clip cup, may be optionally provided to cover at least a proximal portion of the needle tip protector or safety needle clip or simply safety clip and be fixedly attached to the proximal end of the safety clip. The combined safety clip and clip cup thus move in unison. Concentric openings are provided at the proximal end walls of safety clip and clip cup. The openings have a cross dimension smaller than a needle feature such as a protuberance or a bump at the cylindrical wall of the needle cannula. Accordingly, when the needle is moved in the proximal direction relative to the catheter hub, the needle feature eventually would make contact with the openings so that further proximal movement of the needle moves both the needle tip protector and the clip cup proximally, leading eventually to the needle tip protector and the clip cup separating from the catheter hub.

The needle tip protector for the inventive catheter assembly or safety catheter device may have different configurations, so long as there are members that are adapted to be biased by the passage of the needle therethrough and also barrier(s) that prevent the sharp distal tip of the needle from being exposed once it has been withdrawn into the needle tip protector.

The present invention also provides a method of making a safety catheter device according to claim 13.

### Brief Description of the Figures

The present invention will become apparent and the invention itself will be best understood with reference to the following description of the invention taken in conjunction with the accompanying drawings, wherein:
Fig. 1 is a perspective view of an exemplar embodiment of the instant invention safety catheter device;
Fig. 2a is a side view of the Fig. 1 embodiment of the safety catheter device;
Fig. 2b is another perspective view of the Fig. 1 embodiment;
Fig. 3a is a perspective view of the catheter hub of the inventive safety catheter device;
Fig. 3b is a cross-sectional view of the Fig. 3a catheter hub;
Fig. 3c is a side view of the Fig. 3a catheter hub;
Fig. 3d is an end view of the Fig. 3c catheter hub;
Fig. 3e is a front end view of the Fig. 3c catheter hub;
Fig. 4a is a perspective view of the seal member of the inventive safety catheter device;
Fig. 4b is a cross-sectional view of the Fig. 4a seal member;
Fig.4c is a top view of the Fig. 4a seal member;
Fig. 4d is a side view of the Fig. 4a seal member;
Fig. 4e is one end view of the Fig. 4a seal member;
Fig. 4f is another end view of the Fig. 4a seal member;
Fig. 5a is a perspective view of the actuator of the inventive safety catheter device;
Fig. 5b is a cross-sectional view of the Fig. 5a actuator;
Fig. 5c is a side view of the Fig. 5a actuator;
Fig. 5d is one end view of the Fig. 5a actuator;
Fig. 5e is another end view of the Fig. 5a actuator;
Fig. 6a is a perspective view of the needle housing of the inventive safety catheter device;
Fig. 6b is a cross-sectional view of the Fig. 6a needle housing;
Fig. 6c is a top view of the Fig. 6a needle housing;
Fig. 6d is a side view of the Fig. 6a needle housing;
Fig. 6e is an end view of the Fig. 6d needle housing;
Fig. 6f is another end view of the Fig. 6d needle housing;
Fig. 7a is a perspective view of the needle tip protector or clip guard of the inventive safety catheter device;
Fig. 7b is a cross-sectional view of the Fig. 7a clip guard;
Fig. 7c is another cross-sectional view of the Fig. 7a clip guard;
Fig. 7d is a side view of the Fig. 7a clip guard;
Fig. 7e is a proximal end view of the Fig. 7a clip guard;
Fig. 7f is a semi-cross-sectional distal end view f--f of the Fig. 7a clip guard;
Fig. 8a is a perspective view of the flash plug of the inventive safety catheter device;
Fig. 8b is a cross-sectional view of the Fig. 8a flash plug;
Fig. 8c is a side view of the Fig. 8a flash plug;
Fig. 8d is an end view of the Fig. 8c flash plug;
Fig. 8e is another end view of the Fig. 8c flash plug;
Fig. 9a is a perspective view of the needle tip protector cup or clip cup of the inventive safety catheter device;
Fig. 9b is another perspective view of the inventive clip cup;
Fig. 9c is a side view of the Fig. 9a clip cup;
Fig. 9d is cross-sectional view a of the Fig. 9a clip cup;
Fig. 9e is a top view of the Fig. 9a clip cup;
Fig. 9f is an end view of the Fig. 9d clip cup;
Fig. 9g is another end view of the Fig. 9d clip cup;
Fig. 10a is a perspective view of the sheath of the inventive safety catheter device;
Fig. 10b is a cross-sectional view of the Fig. 10a sheath;
Fig. 10c is a top view of the Fig. 10a sheath;
Fig. 10d is a side view of the Fig. 10a sheath;
Fig. 10e is an end view of the Fig. 10d sheath;
Fig. 10f is another end view of the Fig. 10d sheath;
Fig. 11a is a perspective view of an exemplar needle tip protector or safety clip of the inventive safety catheter device;

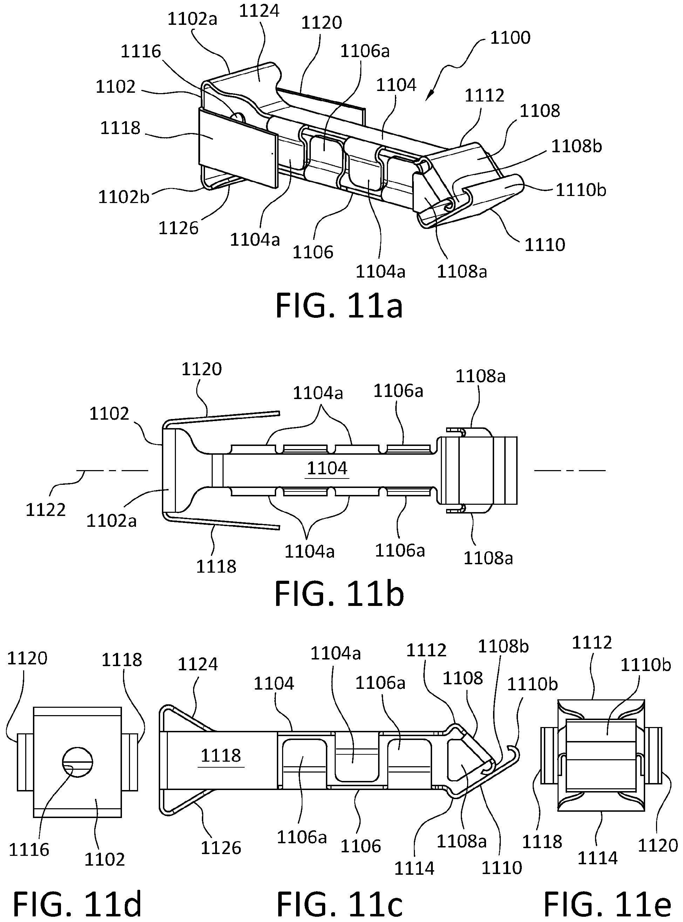

Fig. 11 b is a top view of the Fig. 11a safety clip;
Fig. 11c is a side view of the Fig. 11a safety clip;
Fig. 11d is an end view of the Fig. 11c safety clip;
Fig. 11e is another end view of the Fig. 11c safety clip;

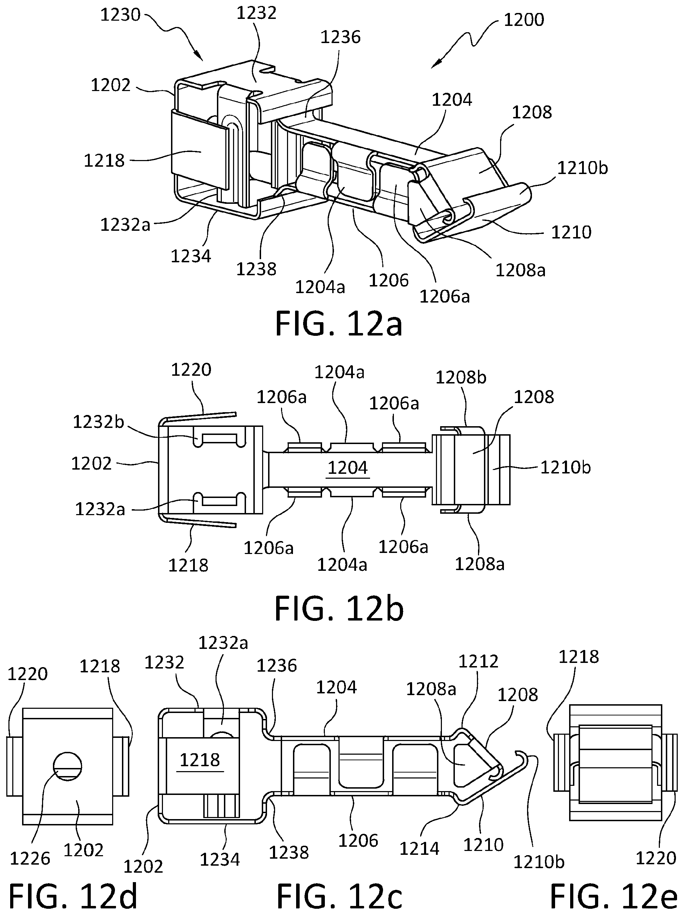

Fig. 12a is a perspective view of another exemplar needle tip protector or safety clip of the inventive safety catheter device with a box shaped proximal portion;
Fig. 12b is a top view of the Fig. 12a safety clip;
Fig. 12c is a side view of the Fig. 12a safety clip;
Fig. 12d is an end view of the Fig. 12c safety clip;
Fig. 12e is another end view of the Fig. 12c safety clip;

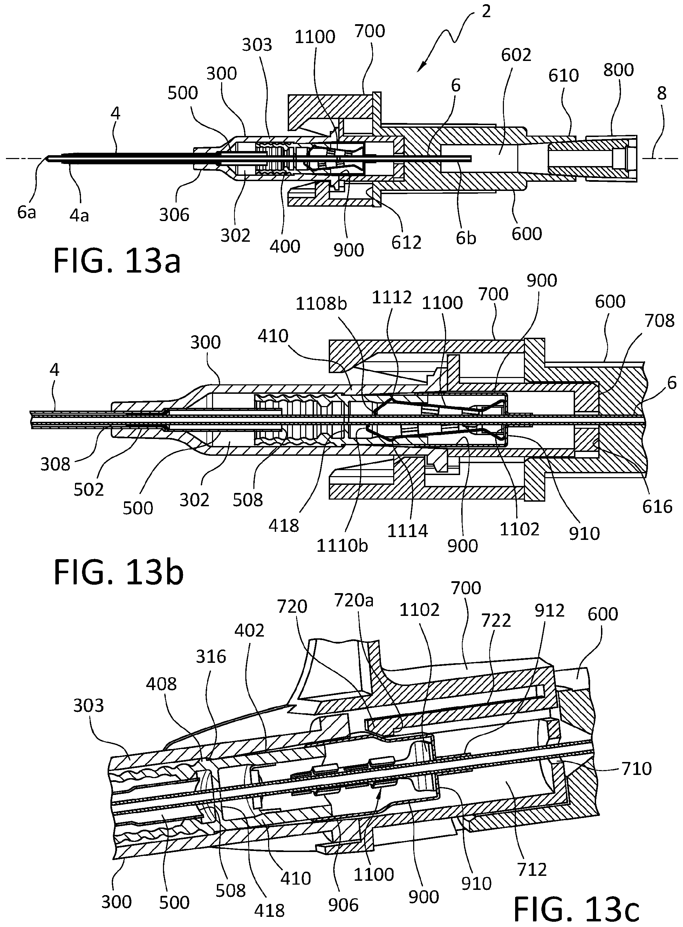

Fig. 13a is a cut-away top view of the inventive safety catheter device in a ready to use position;
Fig. 13b is a cut-away top view of the inventive safety catheter device that includes a clip cup in a ready to use position;
Fig. 13c is a side cut-away view of the Fig. 13b safety catheter device;

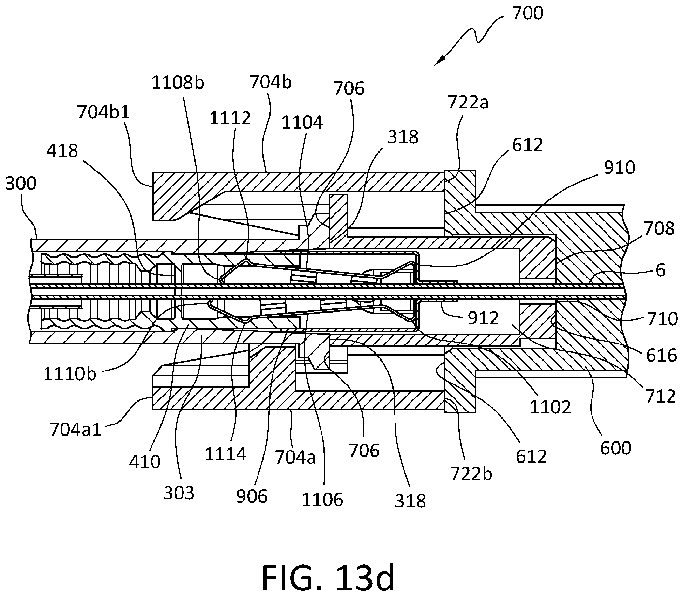

Fig. 13d is an enlarged cross-sectional illustration of the inventive safety catheter device in a ready to use position showing the relationship among the seal member, the needle tip protector, the needle, the clip cup and the clip housing;

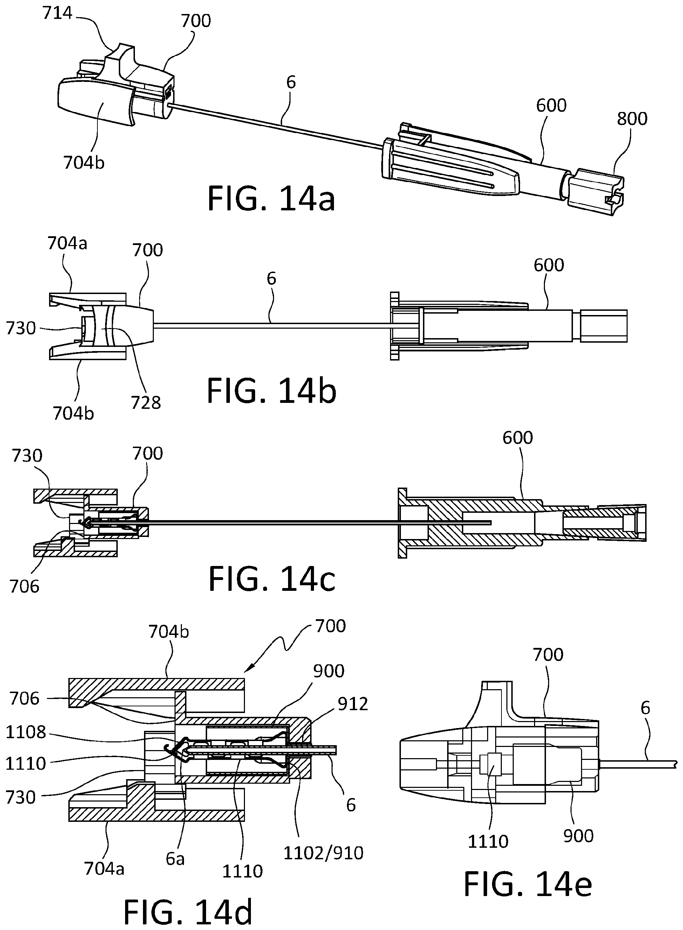

Fig. 14a is a perspective view showing the clip guard and needle housing removed from the catheter hub and in a safe position;
Fig. 14b is a top view of the Fig. 14a clip guard and needle housing;
Fig. 14c is a cross-sectional view of the Fig. 14a clip guard and housing;
Fig. 14d is an enlarged cross-sectional view of Fig. 14c;
Fig. 14e is a semi-transparent and cross-sectional illustration of the needle tip protector guard shown in Figs. 14a-14c;

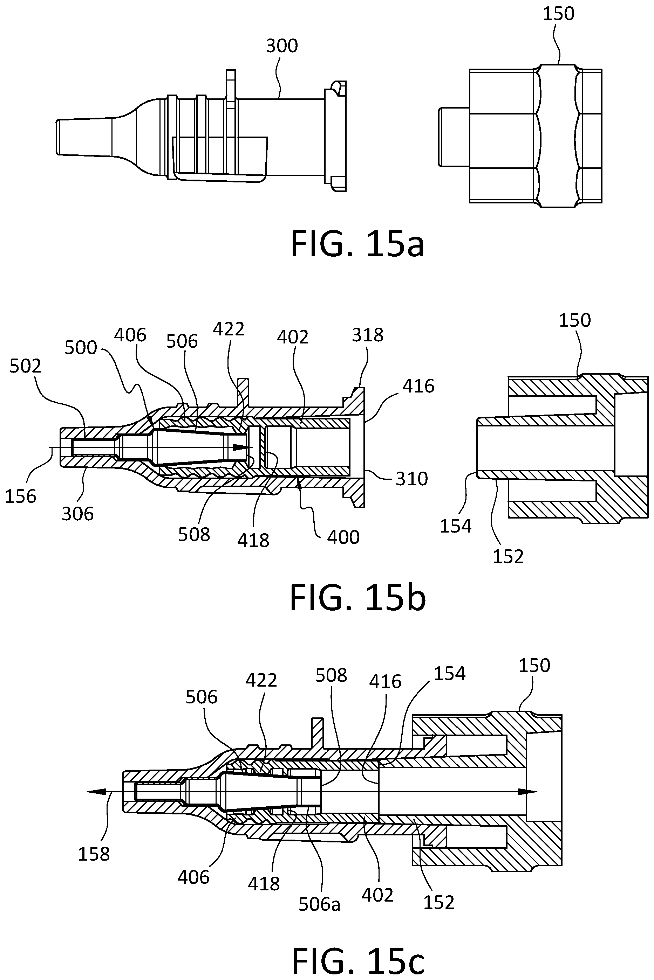

Fig. 15a is a view depicting a catheter assembly and a conventional luer taper connector to illustrate the coupling of a connector to the catheter hub of the inventive catheter assembly after the needle protector clip and clip guard have been removed from the catheter hub;
Fig. 15b is a partial cross-sectional view depicting the catheter hub and luer taper of Fig. 15a showing the membrane of the seal member blocking proximal blood or fluid flow in the interior cavity of the catheter hub;
Fig. 15c is a cross-sectional view depicting the coupling of the luer taper connector to catheter hub, and further showing the tapered nose of the luer connector imparting a distal force against the proximal end of the seal member such that the membrane of the seal member is biased against the proximal opening of the actuator to establish a through fluid path between the connector and the catheter;

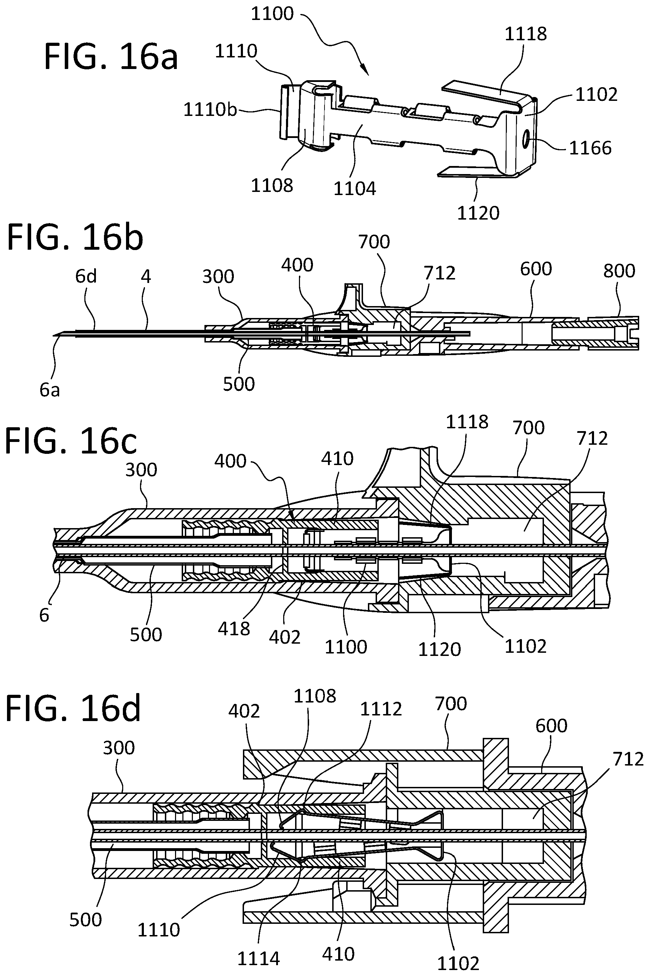

Fig. 16a is an illustration of a different variation of an exemplar needle tip protector or safety clip with angled tangs;
Fig. 16b shows a cross-sectional view of the inventive safety catheter device, or PIVC, with the safety clip of Fig. 11a-11e in the ready to use position;
Fig. 16c is an enlarged cross-sectional view of an of interest portion of the Fig. 16b safety catheter device;
Fig. 16d is an enlarged top view of the of interest portion of the Fig. 16b safety catheter device;

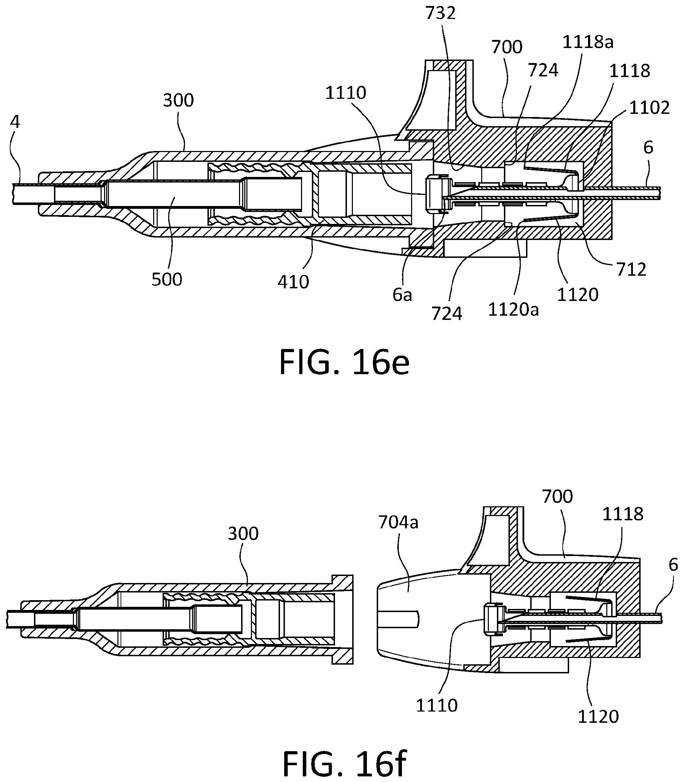

Fig. 16e shows the safety clip having been retracted from the seal member and being withdrawn from the catheter hub;
Fig. 16f shows the clip housing and the safety clip enclosing the distal tip of the needle released from the catheter hub in the safe position;

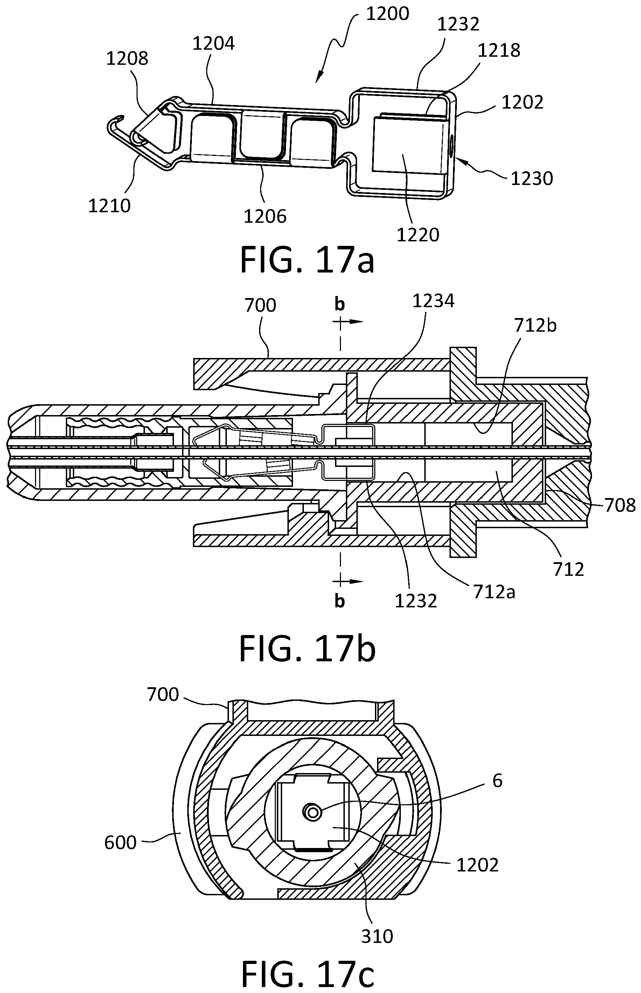

Fig. 17a is a variation of the exemplar box shaped needle tip protector or safety clip of Figs. 12a-12e;
Fig. 17b is a cross-sectional top view of the inventive catheter assembly with the Fig. 17a safety clip in the ready to use position;
Fig. 17c is an end view showing the relationship among the safety clip, the catheter hub and the clip guard;

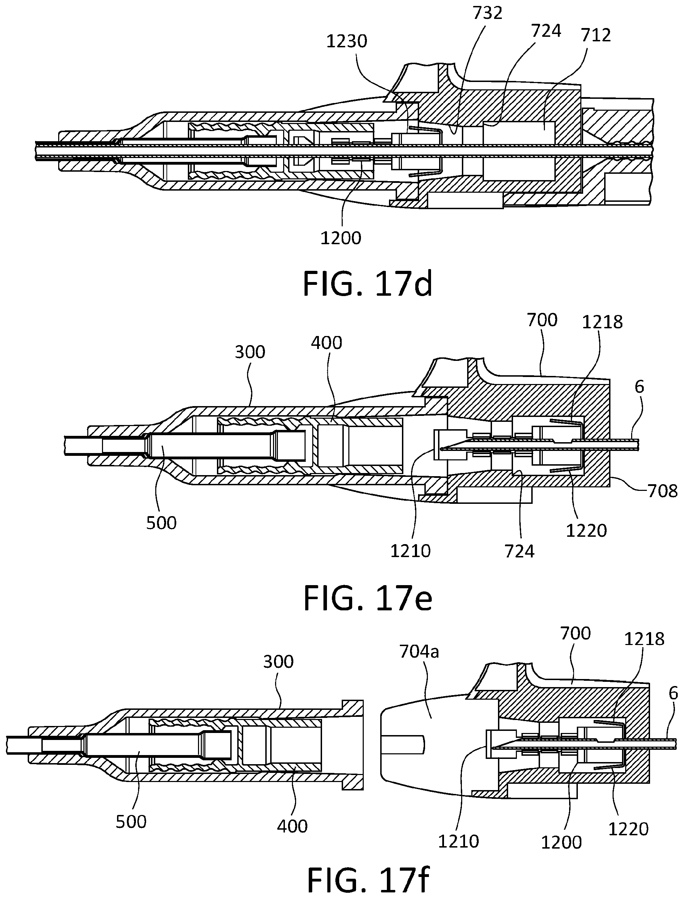

Fig. 17d is a cross-sectional side view of the Fig. 17b catheter assembly;
Fig. 17e is a cross-sectional view showing the safety clip having the distal tip of the needle enclosed therein retracted from the seal member and being withdrawn from the catheter hub;
Fig. 17f shows the clip housing and the safety clip enclosing the distal tip of the needle released from the catheter hub in the safe position;

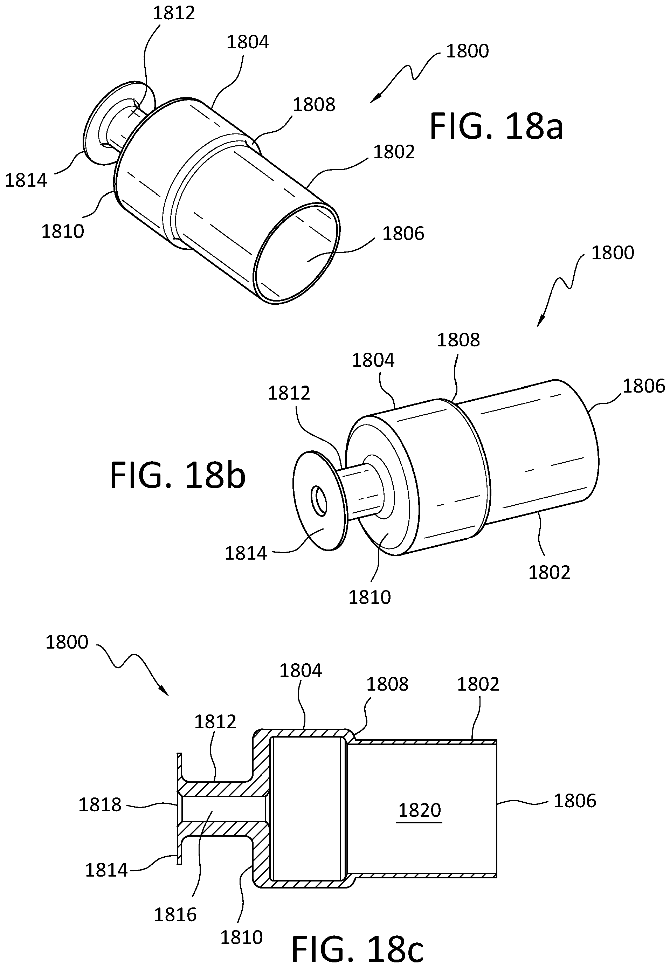

Fig. 18a is a perspective view of the alternative clip cup embodiment;
Fig. 18b is a reverse perspective view of the clip cup of Fig. 18a;
Fig. 18c is a cross-sectional view of the alternative embodiment clip cup;

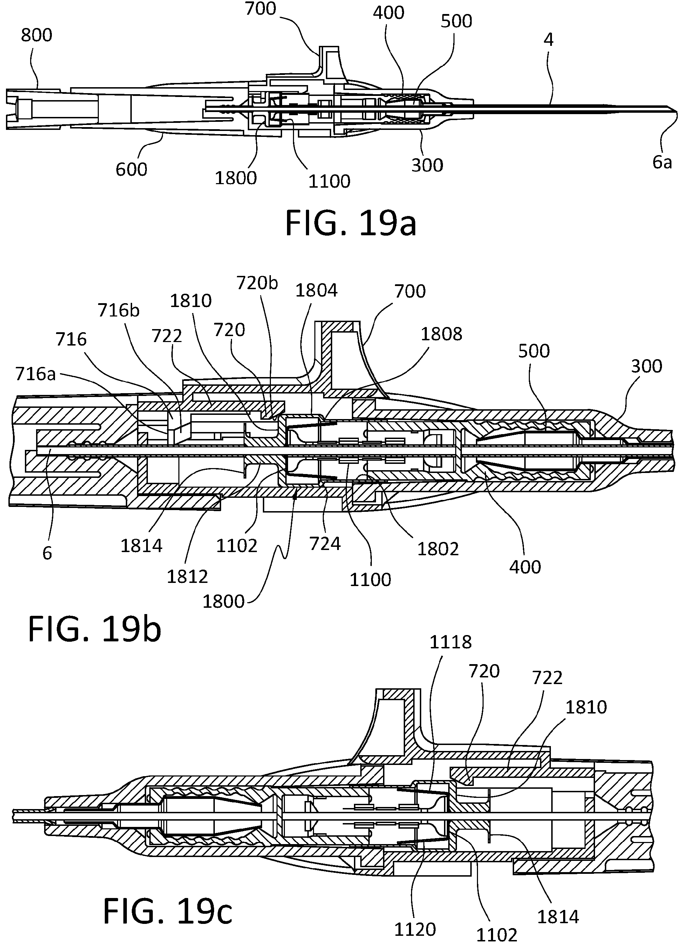

Fig. 19a is a cross-sectional view of the instant invention with an alternate embodiment of the clip cup;
Fig. 19b is an enlarged cross-sectional view of the clip guard and catheter hub shown in Fig. 19a;
Fig. 19c is a reverse cross-sectional view of Fig. 19b;

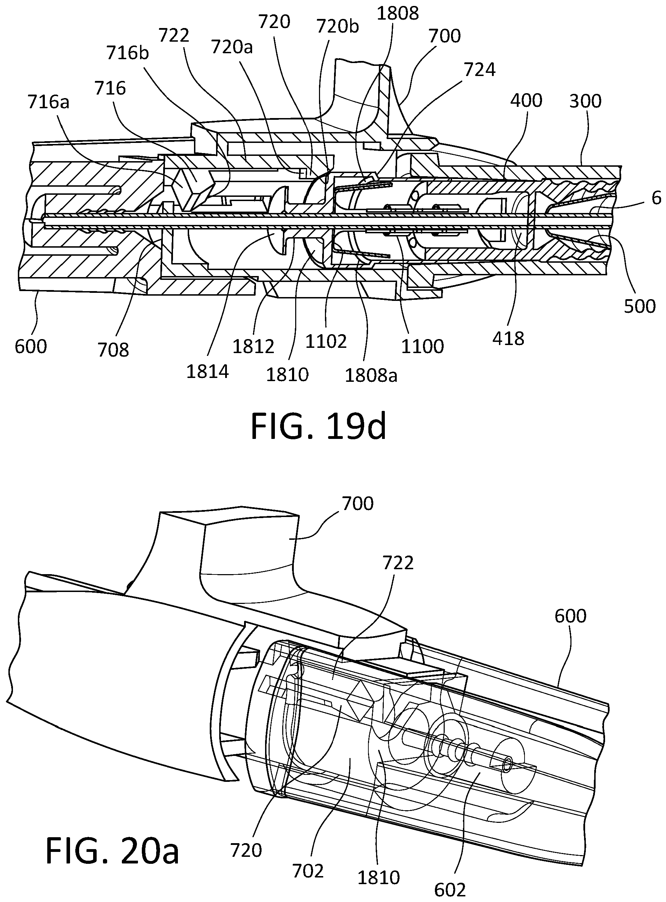

Fig. 19d is a perspective cross-sectional view showing the alternative clip cup and its relationship to the clip guard and the safety clip;
Fig. 20a is a semi-transparent illustration of the needle housing being removed proximally relative to the clip guard;

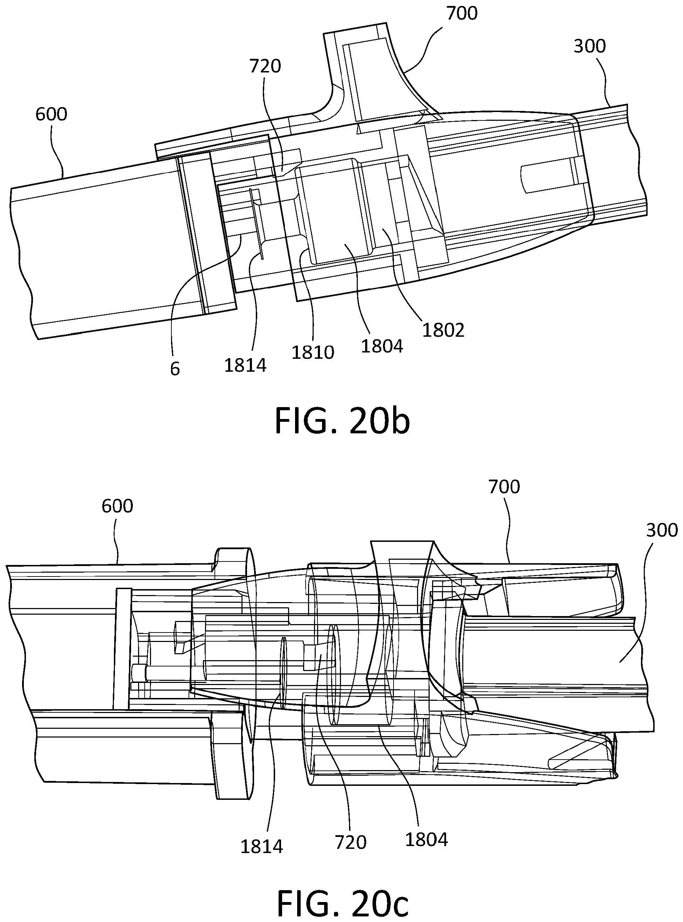

Fig. 20b is a semi-transparent side view showing the needle housing being separated from the clip guard and the relationship of clip cup therewith;
Fig. 20c is a perspective semi-transparent bottom view of Fig. 20a;

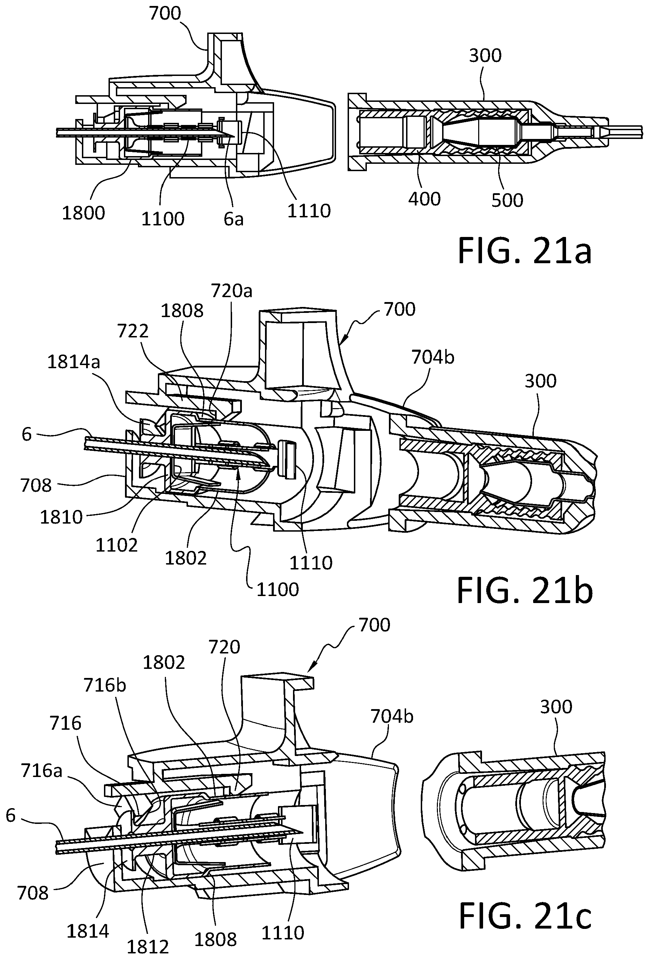

Fig. 21a is a cross-sectional side view showing the safety catheter device in a safe state where the clip cup with the clip cup inside is separated from the catheter hub;
Fig. 21b is a perspective cross-sectional proximal directional view showing the clip cup and safety clip relative to the clip guard having been separated from the catheter hub;
Fig. 21c is a perspective cross-sectional distal directional view showing the clip cup and safety clip relative to the clip guard having been separated from the catheter hub;

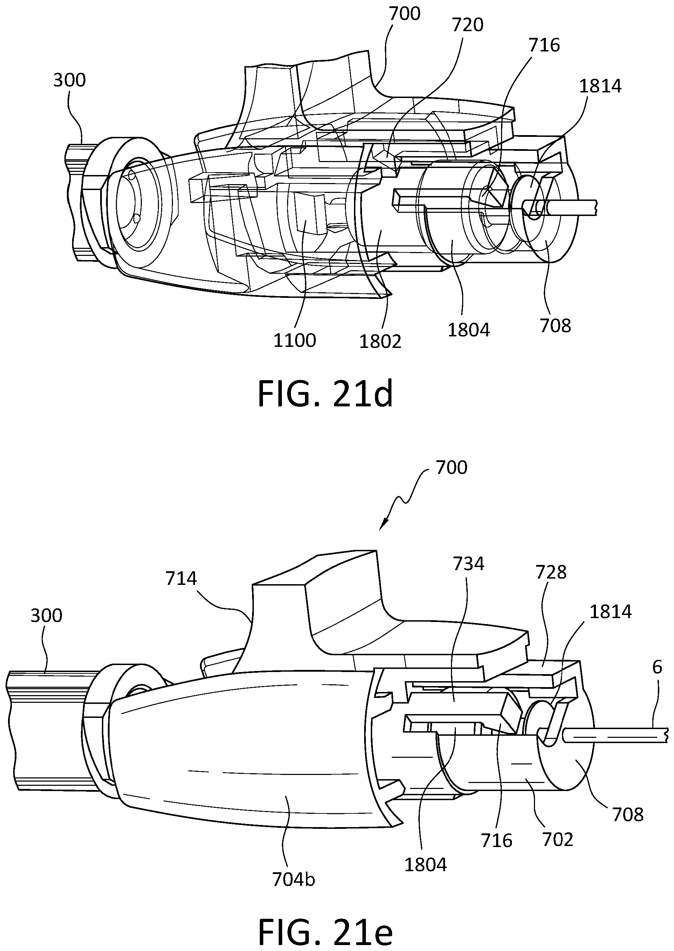

Fig. 21d is a semi-transparent view of the clip guard and clip cup housed therein in the safely locked position; and
Fig. 21e is an illustration showing the safety catheter device in the safe locked state where the clip guard has separated from the catheter hub.

### Detailed Description of the Invention

For the discussion below, it should be appreciated that the term"distal," as used herein, refers to the direction along an axis that lies parallel to a needle cannula of a safety catheter assembly that is closest to the subject during catheter insertion. Conversely, the term"proximal," as used herein, refers to the direction lying along the axis parallel to the needle cannula that is further away from the subject when the catheter is inserted into the vein of the subject, opposite to the distal direction. Other terms that may also be used to refer to the distal end and proximal end of the safety catheter device may include "patient end" and "user end", respectively. Also, for the sake of simplicity, a catheter assembly device, or PIVC, may simply be referred to as "catheter assembly" or "safety catheter", the needle tip protector as "safety needle clip" or simply "safety clip", the needle tip protector cup as "clip cup", and the guard assembly that overlies the proximal portion of the catheter hub and isolates the safety clip from the environment after the safety clip is removed from the catheter hub as "clip guard".

The following US application and patents, assigned to the same assignee as the present application, relate to safety catheters/catheter assemblies: Appl. No. 15/733,439, US 8,652,104, US 10,080,867, US 10,548,522 and US 10,675,440.

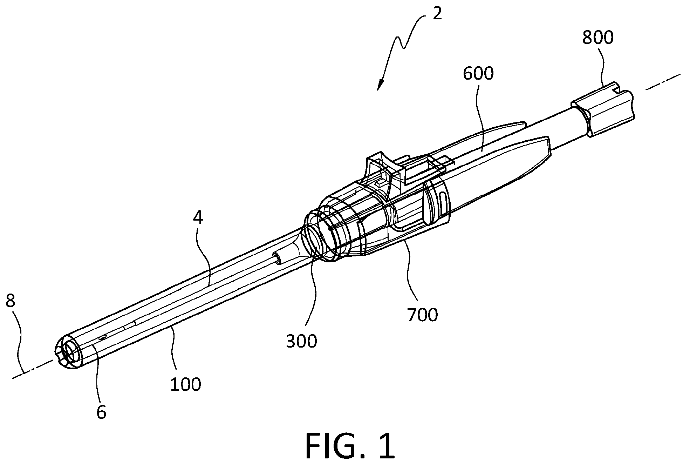

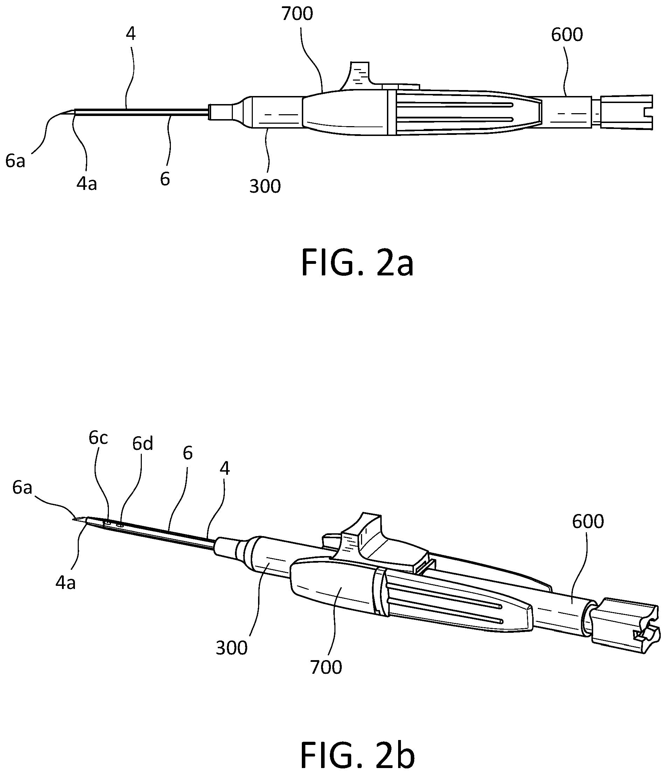

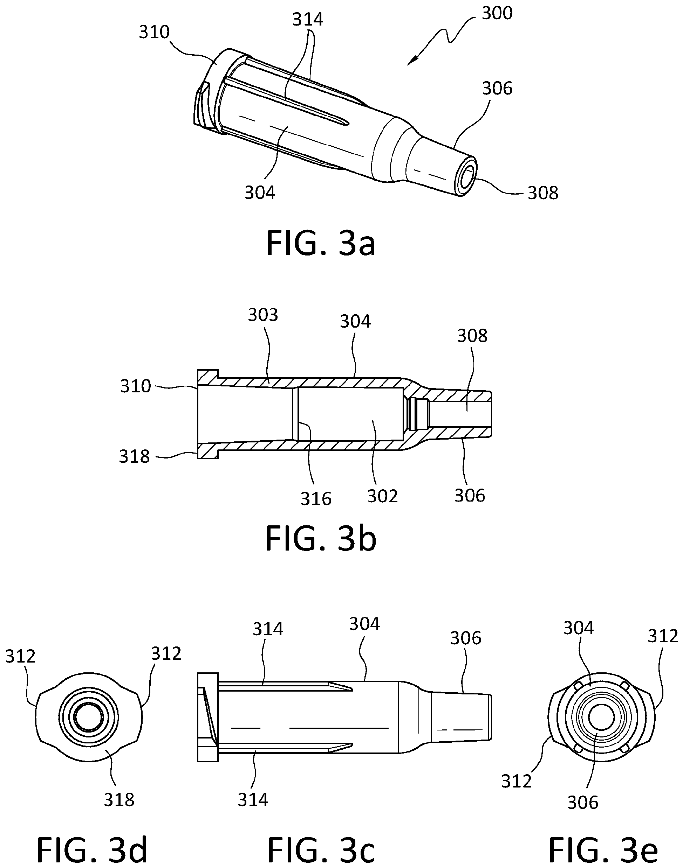

With reference to Figs. 1 and 2a-2b, an exemplar embodiment of the inventive PIVC or catheter assembly 2 of the present invention is shown to include a catheter hub 300, a needle housing 600 and a needle tip protector or clip guard 700. Also shown is a catheter tube or simply catheter 4 that extends from the distal end of the catheter hub 300 to establish a through fluid passageway between the distal end 4a of catheter 4 and the interior cavity (302 in Fig. 3b) as is conventionally known. As shown, catheter assembly 2 extends in alignment along longitudinal axis 8. As best shown in Fig. 13a, a needle 6 extends through the interior cavity of catheter hub 300 to slidably extend internally along catheter 4 such that its sharp distal tip 6a is exposed beyond the distal end 4a of catheter 4. A needle feature or transition 6c, such as for example a bump that may be configured onto the needle cannula in the manner as described in U.S. application No. 16/580,875, is formed on needle 6 proximate to distal tip 6a. The disclosure of the '875 application is incorporated into the disclosure of the present application. A notch 6d may also be provided at the distal portion of needle 6.The proximal end 6b of needle 6 is fixedly attached to a needle hub 602, which is integrally formed inside needle housing 600. It should be appreciated that even though it is shown in the exemplar embodiment, a needle housing may not be necessary for the practice of the invention, as long as there is a needle hub to which the non-patient end of the needle is fixedly attached. As shown in Figs. 1, 2a-2b and 13a, catheter assembly 2 is in a ready to use position.

Catheter hub 300 is shown in Figs. 3a-3e. In detail, catheter hub 300 has a main body portion 304 which wall 303 defines an interior cavity 302, a distal portion 306 having an aperture 308 whereat the proximal end of the catheter 4 is fixedly coupled, and an opened proximal end 310 whereat, as best shown in Figs. 3d and 3f, two threading ribs 312 are provided to enable the catheter hub to be threadingly mated to counterpart connecters of syringes, pumps or the like as is conventionally known. The combined end surfaces of proximal end 310 and the ribs 312 form a proximal contact surface 318. A plurality of splines 314 longitudinally formed or integrated along the outer wall surface of main body portion 304 may be provided to enable a user to more readily grasp the catheter hub. A groove or step 316 may be provided circumferentially about the inner surface of the wall of main body portion 304 to secure a seal member in the interior cavity 302 of catheter hub, as described below.

As shown in Figs. 13a-13d and noted above, a valve or seal member 400 is provided in the interior cavity 302 of catheter hub 300. Seal member 400 is axially shiftable along the interior cavity of catheter hub 300. Seal member 400 may be made from an elastomeric material that is generally flexible and may include suitable materials such as, for example, silicone or polyisoprene. In one embodiment, the seal member 400 may be formed as a unitary or monolithic member through various molding processes including, for example, injection molding processes generally known in the art.

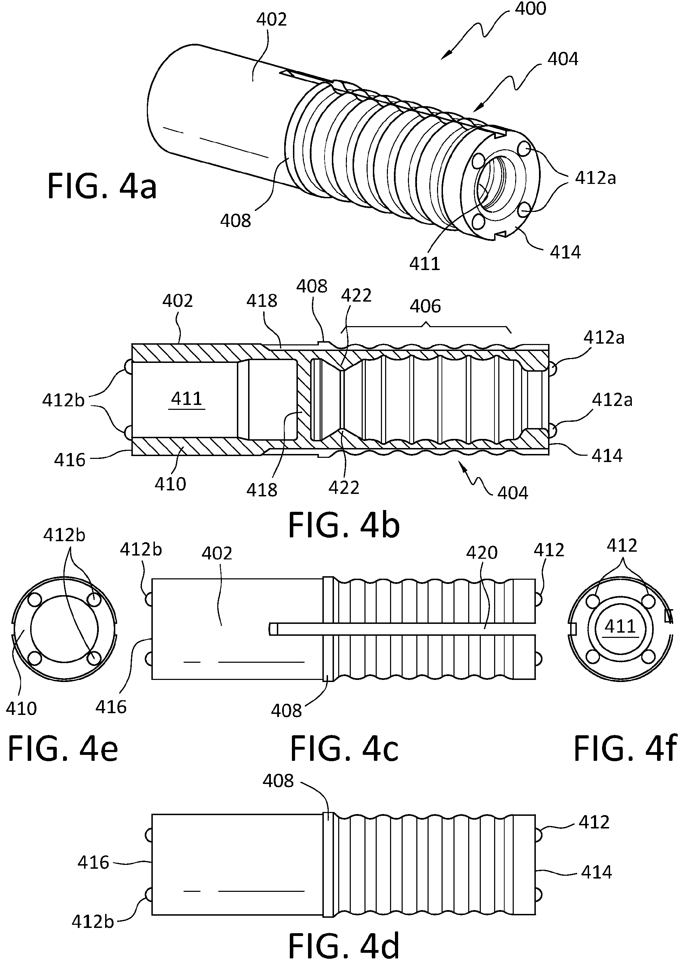

With reference to Figs. 4a-4f, seal member 400 has a substantially cylindrical shaped wall 410 that defines a bore 411. Seal member 400 is shown to include a proximal cylindrical portion 402 and a distal portion 404. The portion of wall 410 that defines proximal portion 402 is substantially uniform along its length, whereas the portion of wall 410 that defines distal portion 404 has a corrugated shape and may be in the shape of an accordion along length 406. An integral membrane 418 partitions bore 411 between proximal portion 402 and distal portion 404. There are a number of projections 412a at the distal end 414 and a similar number of projections 412b at the proximal end 416 of the seal member 400 that act as contact points for the respective distal and proximal end surfaces 414, 416 of the seal member. A groove 420 is formed along a substantial portion of seal member starting from its distal end 414 to provide an air escape path during actuation of seal member, as will be described. When fitted into the interior cavity 302 of catheter hub 300, given that the seal member 400 is made of an elastomeric material, accordion shaped distal portion 404 is compressible when proximal portion 402 is under pressure or is impacted by a distal driving force at proximal end 416 and would return to its natural position when the pressure or impact force is removed from proximal end 416. Although shiftable axially, seal member 400 is non-removably secured to the interior cavity 302 of catheter hub 300, as a circumferential flange 408 at approximately the juncture between proximal portion 402 and distal portion 404 catchingly engages the step 316 at the interior cavity 302 of catheter hub 300. Step 316 prevents seal member 400 from proximal movement out of catheter hub 300 once seal member 400 is fittingly inserted and seated into the interior cavity 302 of catheter hub 300 by way of opened proximal end 310 of catheter hub 300.

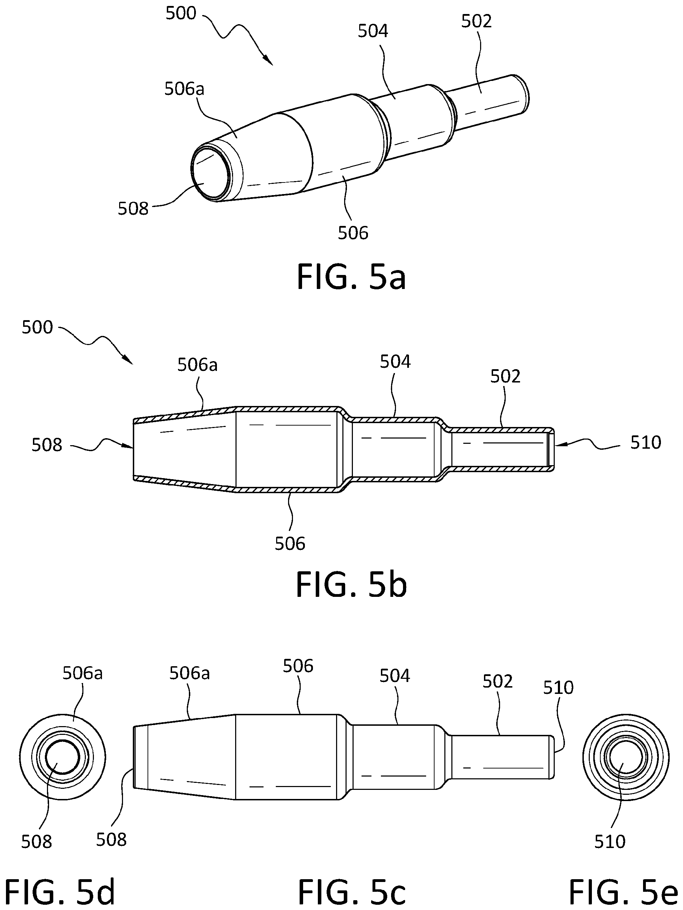

As further shown in Figs. 13a-13d, provided inside interior cavity 302 and distal of membrane 418 is an actuator. An exemplar actuator which may not necessarily resembles that shown in Figs. 13a-13d is illustrated in Figs. 5a-5e. Note that the actuator shown in Figs. 13a-13d is similar to the actuator shown in Fig. 19 of the aforenoted incorporated by reference '104 US patent. The actuator 500 shown in Figs. 5a-5e is an improved actuator that may be used in the inventive safety catheter device so as to replace the actuator shown in Figs. 13a-13d. For the sake of simplicity, the actuator in Figs. 13a-13d and the actuator shown in Figs. 5a-5e are both designated by the reference number 500.

With reference to Figs. 5a-5e, the exemplar actuator 500 is shown to have a distal portion 502, an intermediate portion 504 and an enlarged portion 506 that has a frusto-conical portion 506a that ends with an opened proximal opening 508. Portions 506 and 506a may be considered as a single frusto-conical portion. Thus, the diameter of opening 508 is smaller than the diameter across portion 506. Distal portion 502 has a distal opening 510, such that a through passage extends between distal opening 510 and proximal opening 508 for actuator 500. By having frusto-conical section 506a, membrane 418 of seal member 400 may be more readily pierced or opened when seal member 400 is driven to shift axially in the distal direction by an impact force such as the luer connector of a syringe or other similar connector, as described in the '104 US patent.

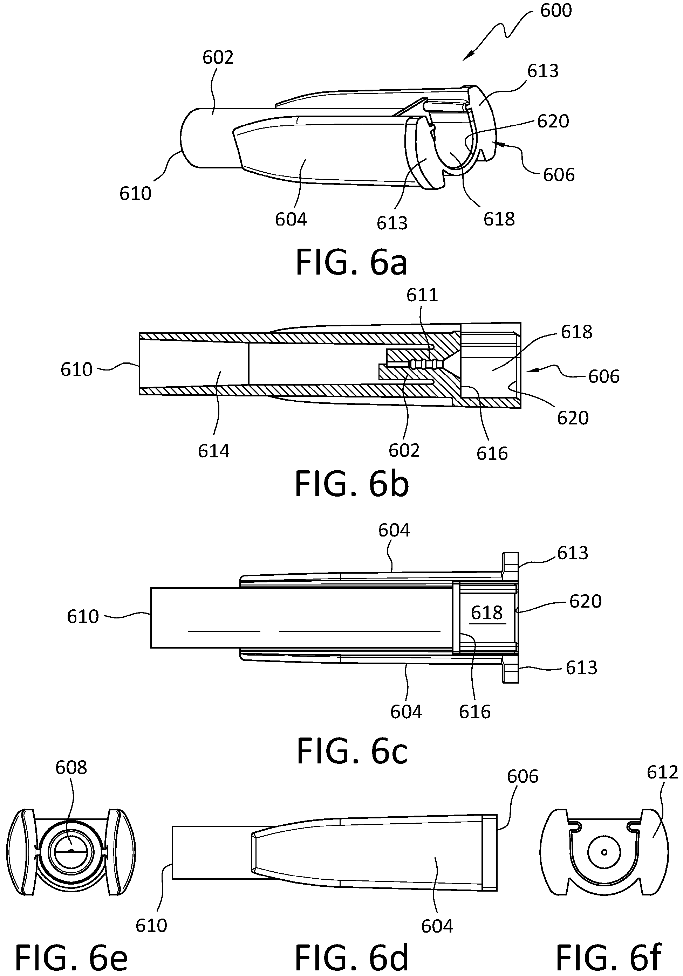

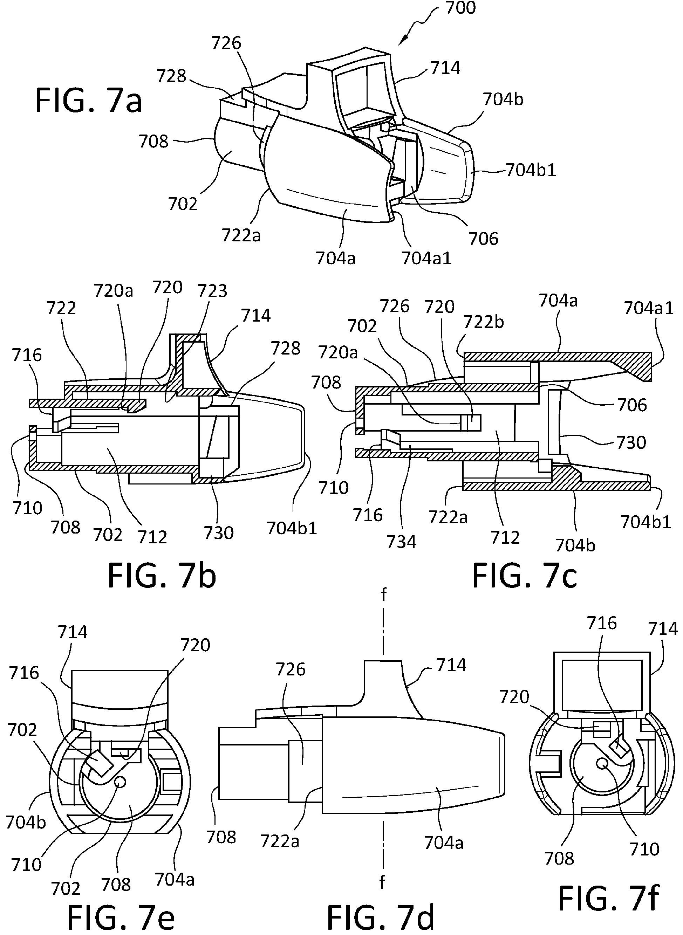

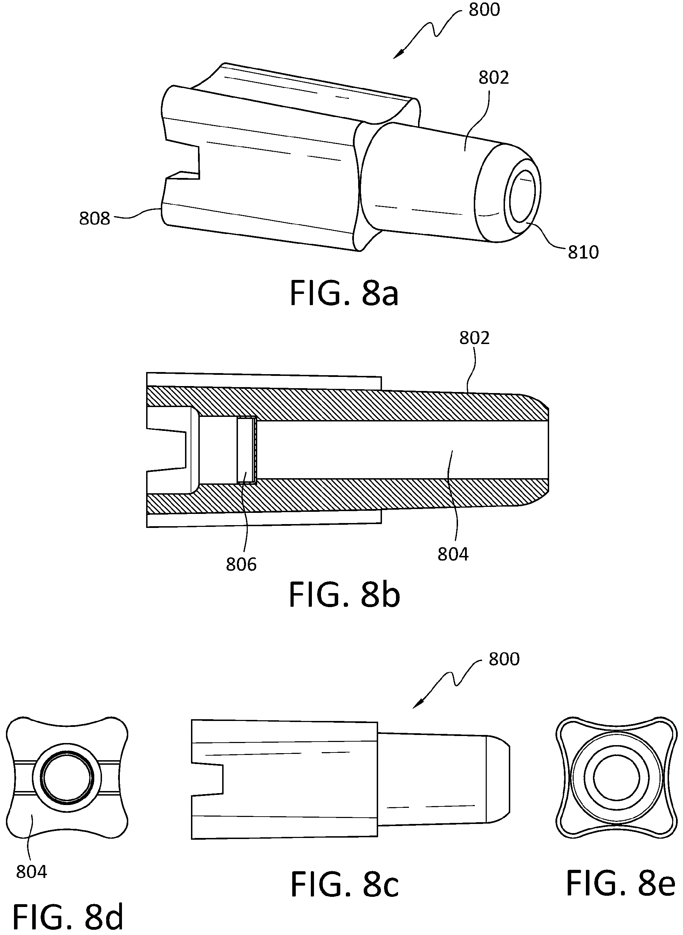

With reference to Figs. 6a-6f, the needle housing 600 of the inventive safety catheter device is shown to include a housing 602 that has two side gripping portions, or simply grippers 604 to enable the user or clinician to readily grasp the needle housing. A flash chamber 614 has a proximal opening 610 adapted to receive a flash plug 800, as shown in Figs. 8a-8e. Fig. 6b shows a needle hub 602 in needle housing 600 having a slot 611 configured to accept the proximal end 6b of needle 6 in the manner as per shown in Fig. 13a. Slot 611 fixedly holds the proximal end 6b of needle 6. A bore 618 that is opened at the top, as shown in Fig. 6a, is configured to accept the cylindrical portion 702 of the clip guard shown in Figs. Fig. 7a. A rib 620 is formed at the distal end 606 of bore 618. Fig. 6f shows the interconnected proximal end surfaces 613 of the side grippers of needle housing 600.

The needle tip protector guard, or simply clip guard 700, is shown in Figs. 7a-7f. Clip guard 700 has a cylindrical portion 702 that has two side extensions, wing covers or shields 704a and 704b integrally extending from the cylindrical portion 702. In particular, a distal portion of each of side wing shields 704a and 704b extends beyond distal end 706 of cylindrical portion 702. Conversely, the proximal portion of cylindrical portion 702 is not covered by the side shields 704a and 704b. Although two wing shields are shown, it should be appreciated that one continuous shield or a plurality of more than two shields may also be used to provide coverage for the inventive clip guard. At the back wall or proximal end base 708 of cylindrical portion 702 there is an aperture 710 to enable a needle cannula to pass into the interior chamber 712 of cylindrical portion 702, and from there extends out of distal end 706 of cylindrical portion 702, as per shown by needle 6 in Figs. 13a-13d. A top cover 728 and a lower cover 730 extend distally from distal end 706. An upright 714 at the top surface of clip guard 700 provides a thumb or finger rest for the user or clinician in the handling of the safety catheter. Tapered surfaces 726 at cylindrical portion 702 enable the needle housing 600 to removably couple to clip guard 700 by the frictional interaction of the tapered surfaces 726 and the rib 620 at needle housing 600. As shown in the cross-sectional view of Fig. 7b and the end view of Fig. 7e, the top of cylindrical portion 702 merges with upright 714. A finger hook 720 is at the distal end of a cantilever beam 722 that extends from proximal end base 708. Although not necessary for the operation of the safety catheter device shown in Figs. 13a-13d, an internal shoulder 723 may be provided at interior chamber 712 and a catch stop 716 may be provided proximate to proximal end base 708 of cylindrical portion 702 for use with different needle tip protector and/or clip cup, to be described, infra. Clip guard may be made from a medical grade plastic material such as for example acrylonitrile butadiene styrene (ABS). The wall at proximal end base 708 of the clip guard 700 has a partial opening as per shown in Figs. 7b and 7e. This opening may be needed for the formation of finger hook 720 and catch stop 716 during the molding process of clip guard 700. It should be appreciated however that a clip guard that has an enclosed proximal end wall with only an opening for the needle to pass through may also be used.

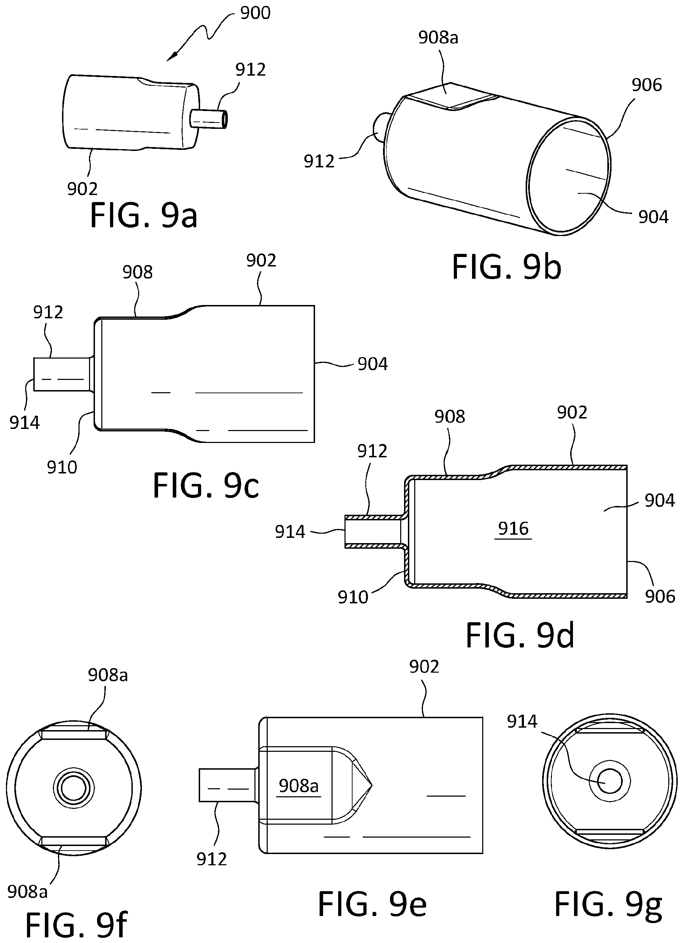

Figs. 8a -8e show flash plug 800 that is inserted into the proximal opening 610 of needle housing 600 described in Figs. 9a-6f. Flash plug 800 has a distal cylindrical portion 802 adapted to fit into the proximal opening 610 of needle housing 600. Thus fitted, blood or other fluid from the needle would be drawn into flash chamber 614 of needle housing 600, with air in the chamber passing into the passage 804 of plug 800. An air permeable hydrophilic filter 806 in passage 614 allows air in passage 804 to pass but prevents fluids such as blood from passing. The wall of flash chamber 614 is transparent or translucent and therefore may be viewed by the clinician for the presence of blood, which when seen, is an indication that the needle is correctly entered into the vein or artery of the patient. The respective end views of the proximal end 808 and the distal end 810 of flash plug 800 are shown in Figs. 8d and 8e, respectively.

Further included in the safety catheter device of the instant invention is a needle tip protector cup, or simply clip cup 900, as per shown in Figs. 9a-9f. Clip cup 900 is in the form of an open ended cylinder that has a distal portion 902 which has an opening 904 at its distal end 906. Cup 900 may be made of metallic material or a hard medical grade plastic. Distal portion 902 is shown to taperedly merges into a proximal portion 908 that has top and bottom opposing substantially flat surfaces 908a. Distal portion 902 has a diameter that is larger than the diameter of proximal portion 908. A hollow shaft 912 extends from proximal base 910 and is configured to have an opening 914 that has a cross section that allows a needle cannula such as needle 6 to pass through into the interior cavity 916 of clip cup 900 but prevents the above discussed needle feature or transition from passing. Opposing flat surfaces 908a may act as guides to align clip cup 900 within chamber 712 of clip guard 700.

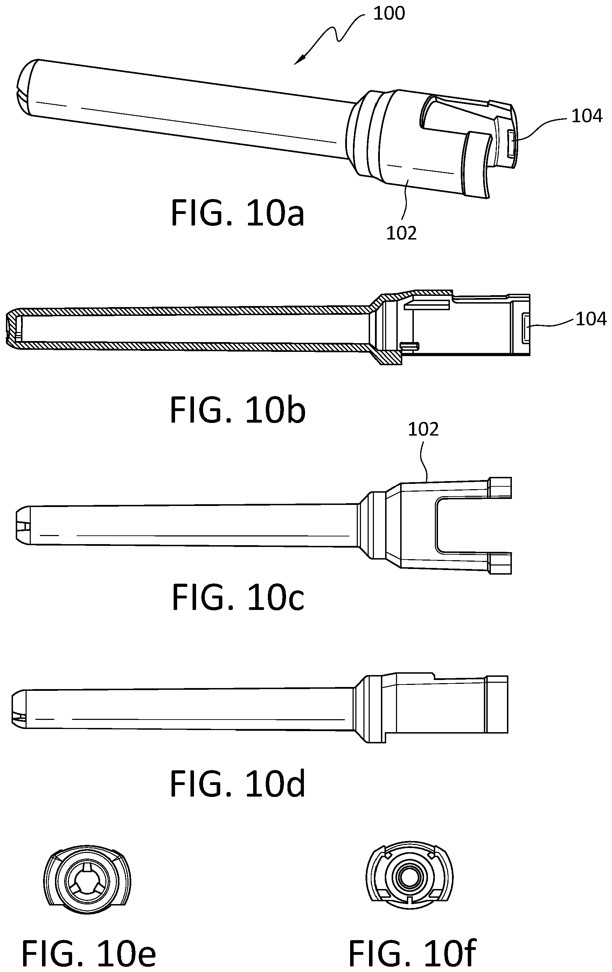

Figs. 10a-10f show different views of an exemplar sheath 100 that covers the catheter 4 and the needle 6 slidably extending therealong, as per shown in Fig. 1. The base 102 of sheath 100 is configured such that it is adapted to superposed over catheter hub 300 and be in removable contact with clip guard 700 in the manner shown in Fig. 1 to protect the needle and catheter for shipping and before the safety catheter device is used. Inward facing flanges 104 at the proximal end of base 102 may be used to frictionally or snap hold sheath 100 to clip guard 700 or the catheter hub 300 in a conventional manner. Sheath 100 is removed when the safety catheter device is ready to be used.

A first embodiment of a needle tip protector or needle protector clip, or simply safety clip 1100, is shown in Figs. 11a-11e. Needle tip protector or safety clip 1100 has a proximal base 1102 that has two elongated arms 1104 and 1106 connected thereto by bridging portions 1124 and 1126, respectively. Bridging portions 1124 and 1126 each narrow like the top half of an hour glass, or Y-shaped, from the opposing side edges of base 1102 to the respective arms 1104 and 1106 such that the arms are configured to extend substantially orthogonally from base 1102 but at planes that are sandwiched by the imaginary planes of the opposing sides that extend coplanarly as the arms. Thus, arms 1104 and 1106 extend along respective parallel planes that are below the opposing sides of base 1102 of safety clip 1100, as per shown in Figs. 11a-11e. The Y-shape configuration of portions 1124 and 1126 provide more flexibility for arms 1104 and 1106. Arms 1104 and 1106 are substantially in parallel to each other when in their natural or unbiased position.

Arms 1104 and 1106 each have side ribs 1104a and 1106a, respectively, that extend along the respective longitudinal sides of the arms. Ribs 1104a and 1106a are configured to be substantially orthogonal toward the longitudinal central axis of the safety clip 1100. When in the position as shown in Figs. 11a-11c, arms 1104 and 1106 together with their respective ribs 1104a and 1106a form a cage like enclosure. Barriers 1108 and 1110 are formed at the respective distal ends of arms 1104 and 1106, with distal end barrier 1110 overlapping distal end barrier 1108 when the safety clip is in a closed or safe position. Barrier 1108 has side ribs or partitions 1108a to protect the sharp distal tip of the needle when the distal portion of a needle is withdrawn and enclosed in safety clip 1100, as the distal tip of the needle is encircled by the distal end barriers and the side partitions 1108a. It should be appreciated that side partitions may be provided at barrier 1110 instead of at barrier 1108.

Barriers 1108 and 1110 have respective curved distal end or fingers 1108b and 1110b. Rounded apexes, projections, protrusions or bosses 1112 and 1114 are formed at the junction where arms 1104 and 1106 connect to their corresponding barriers 1108 and 1110. An opening 1116 is formed at base 1102 that has a diameter sized to enable a needle cannula such as needle 6 to pass through but prevents the needle feature such as 6c to pass. The needle is adapted to pass between the one and other distal end barriers when the barriers are positioned in an interior cavity of the catheter hub such that the barriers are biased in opposing directions away from the central axis to effect a non-removable engagement with the catheter hub in a ready to use position. Further extending from base 1102 at an angle away or offset from the longitudinal central axis of safety clip 1100 are two substantially rigid projecting shanks, prongs or tangs 1118 and 1120. The safety clip may be made from a metallic material such as stainless steel but with sufficient flexibility so that arms 1104 and 1106 may be biased scissor like from base 1102 in a direction away the longitudinal axis of the safety clip, when a needle passes through and biases fingers 1108b and 1110b away from each other. Tangs 1118 and 1120, on the other hand, are flexibly moved toward each other when restricted or when they pass through a restricted area, and will return to their outwardly angled natural position when no longer restricted.

An alternative needle tip protector, or safety clip1200, is shown in Figs. 12a-12e. For safety clip 1200, components that are the same or function in the same manner as safety clip 1100 shown in Figs. 11a-11e are labeled the same, except the first two digits of the components are changed from "11" to "12", so that the components for the safety clip in Figs. 12a-12e are labeled "12xx" instead of "11xx". Safety clip 1200 differs from safety clip 1100 in that the proximal portion 1230 of safety clip 1200 has a box like structure that has an upper plate 1232 and a lower plate 1234 respectively extending in the distal direction orthogonally from proximal end wall or base 1202 that has an opening 1226 at the center of the proximal end wall. Upper plate 1232 and lower plate 1234 are in parallel to each other. Supports 1232a and 1232b downwardly extend from upper plate 1232 rest onto bottom plate 1234 to provide structural rigidity for the box like proximal portion 1230. Upper and lower arms 1204 and 1206 are integral extensions from upper and lower plates 1232 and 1234, respectively, that have corresponding curved bent or bridging portions 1236 and 1238. Thus, upper and lower arms 204 and 1206 extend along respective in parallel planes that are sandwiched by coplanar planes of the upper and lower plates 1232 and 1234 when the arms are in the safe position. Although the terms "upper" and "lower" are used to reference the plates, it should be appreciated that the plates may also be referenced as "one" and "other" plates, or "first" and "second", or other terms that would distinguish the plates being different. It should further be appreciated that the elongated arms of the safety clip as shown in Figs. 11a-11e and 12a-12e likewise may be referred to as "upper" and "lower", "one" and "other", or "first" and "second", and other equivalent terms well known in the patent drafting terminology.

It should be appreciated that the needle tip protector or safety clip as described above may be used in other safety catheter devices that do not require bosses of the safety clip to engage a seal member. For example, the described needle tip protectors or safety clips may be used with catheter hubs where the bosses may be biased by the needle into engagement with an internal groove or counterpart locking mechanism at the inner wall of the catheter hub, or used in a closed catheter system where the safety clip is positioned proximal of the septum in the interior cavity of the catheter hub. So, too, when used with the clip cup as described in this disclosure, the combined needle tip protection device may be used with different safety catheter devices or catheter assemblies, with or without seal member or septum, to secure the presumably contaminated sharp distal tip of the needle after use.

In Figs. 13a-13d, the safety catheter device incorporates the clip cup 900 shown in Figs. 9a-9g and the safety clip 1100 shown in Figs.11a-11e minus the tangs. To keep things simple, the safety clip and its components shown in Figs. 13a-13d are designated with the same reference number as the safety clip 1110 and its same components shown in Figs. 11a-11e. Further, it should be noted that the actuator shown in Figs. 13a-13d, although adapted to perform the same functions, has some structural difference from the actuator shown in Figs. 5a-5e. Thus, the components of the actuator that perform or have the same functions as the actuator shown in Figs. 5a-5e are labeled with the same reference numbers. Furthermore, for the embodiment of the safety catheter device shown in Figs. 13a-13d, catch stop 716 is not needed and therefore is removed from the clip guard 700 used with the safety catheter device.

The safety catheter device in Figs. 13a-13d is in its ready to use position. As shown, catheter 4 extends distally from the distal portion 306 of catheter hub 300. As described int the aforenoted '104 US patent, catheter 4 is fictionally held to the bore at distal portion 306 by the distal portion 502 of actuator 500. Once distal portion 502 of actuator 500 is firmly seated into bore 308 of distal portion 306 of catheter hub 300, the proximal end of actuator 500 is positioned distal to membrane 418 of seal member 400. Seal member 400 is shiftable axially along the longitudinal axis 8 but is non-removably fixed to the inner circumferential surface of catheter hub 300. Membrane 418 partitions the accordion portion 404 from proximal portion 402.

In the ready to use position, for the inventive safety catheter device, the distal portion of the safety clip 1100 is inside the proximal portion 402 of seal member 400. Since needle 6, at the ready to use position, extends through the safety clip 1110, the distal fingers 1108b and 1110b of arms 1104 and 1106, respectively, are biased by needle 6 in a direction away from longitudinal axis 8. As a result, bosses 1112 and 1114 of arms 1104 and 1106, respectively, are pressed onto the inner surface of wall 410 of seal member 400 such that safety clip 110 is non-removably attached to seal member 400. Seal member 400 in turn is in non-removable friction contact with catheter hub 300 due to wall 410 of seal member 400 being pressed by bosses 1112 and 1114 against wall 303 of catheter hub 300. As the length of needle 6 is such that its distal tip 6a extends beyond the distal end 4a of catheter 4 and its proximal end attached to needle hub 602 in needle housing 600, the configuration of the safety catheter device 2 is as per shown in Figs. 13a-13d in the ready to use position.

Further with reference to Figs. 13a-13d, needle protection guard or clip guard 700 is shown to be in coupling relationships with both catheter hub 300 and needle housing 600. In particular, in the proximal direction, cylindrical portion 702 of clip guard 700 is accepted into bore 618 of needle housing 600 such that the surface of the proximal end base 708 of clip guard 700 is in contact with the distal surface 616 of needle housing 600, and the surface 612 at the distal end 606 of needle housing 600 are in contact with the proximal end 722a and 722b of side shields 704a and 704b, respectively. Distally, the surface at distal end 706 of clip guard 700 is in contact with the proximal end surface 308 of catheter hub 300. Shields 704a and 704b extend in the distal direction so that their respective distal edges 704a1 and 704b1 would extend to the position as shown in Figs. 13a-13d to substantially cover or shield the proximal half of catheter hub 300.

Clip cup 900 is positioned within chamber 712 of clip guard 700, with its distal end 906 and a distal part of the distal portion 902 circumferentially fitted or inserted between a portion at the proximal end 416 of seal member 400 and the inner wall of catheter hub 300. The distal part of distal portion 902 of clip cup 900 is therefore sandwiched and frictionally held by the respective proximal end portions of catheter hub 300 and seal member 400 at the ready to use position. This holding of the distal end portion of clip cup 900 helps to stabilize clip cup 900 and safety clip 1100 in chamber 712 of clip guard 700. It should be appreciated that clip cup 900 also acts to stabilize safety clip 1100 in the catheter hub even when the seal member 400 is not in the catheter hub or when distal end 906 of cup 900 does not make contact with the seal member, since distal end 906 nonetheless is in friction contact with the inner wall of catheter hub 300 so that distal portion 902 of cup 900 rests onto the inner wall of catheter hub 300. Sandwiching the distal part of distal portion 902 between the inner wall of catheter hub 300 and seal member 400 is preferable to prevent possible flexing of the distal portion of cup 900.

The base 1102 of safety clip 1100 and base 910 of clip cup 900 are fixedly attached to each other by any one of conventional known methods such as welding, bonding, gluing, soldering and/or by using any one of different adhesives means. Thus, clip cup 900 and safety clip 1100 move in unison. As shown in Fig. 9g, an opening 914 is provided at the hollow tube or shaft 912 to enable needle 6 to pass therethrough. Opening 914 at clip cup 900 and the opening 1106 at base1102 of safety clip 1100 are in alignment and the cross dimension of the openings is such as to prevent the needle feature or transition 6c (Fig. 2b) from passing through, when needle 6 is withdrawn from catheter 4 and moved in the proximal direction relative to catheter hub 300. In the ready to use position as shown in Figs. 13-13d, base 910 of clip cup 900 is distal from the proximal end base 708 of clip guard 700. An opening 710 at proximal end base 708 of clip guard 700 has a cross dimension that allows tube 912 of clip guard 900 to pass into.

In use, the user or clinician can enter the vasculature of the patient by means of sharp distal tip 6a so that distal end 4a of catheter 4 may be placed inside the desired vein or artery of the patient. Upon detection of blood in the flash chamber 800 which confirms the correct vasculature placement of distal tip 6a of needle 6 and therefore catheter 4, the user would move needle housing 600 in the proximal direction relative to catheter hub 300 so as to withdraw needle 6 from catheter hub 300. When the distal tip 6a of needle 6 is withdrawn past fingers 1108b and 1110b of safety clip 1100, since they are no longer being biased away from each other, arms 1104 and 1106 of safety clip 1100 would moved toward the longitudinal axis 8 so as to return to their natural position with barriers 1108 and 1110 closing jaw like over the contaminated sharp distal tip 6a of needle 6, as per shown in Figs. 11a-11c. Distal tip 6a of needle 6 is thus enclosed inside safety clip 110 isolated from the environment and prevented from reemerging distally from safety clip 1100.

With arms 1104 and 1106 returning to their natural position, the bosses 1112 and 1114 are no longer engaged to the wall 410 of seal member 400. Further movement in the proximal direction relative to catheter hub 300 causes needle feature 6c to come into contact with the concentric openings at base 1102 of safety clip 1100 and base 910 of clip cup 900. Since needle feature 6c has a larger dimension than the concentric openings, further proximal movement of needle 6 withdraws safety clip 1100 and clip cup 900 in the proximal direction into chamber 712 of clip guard 700 until base 910 of clip cup 900 makes contact with proximal end base 708 of clip guard 700. Thereafter, further proximal movement of needle housing 600 to move needle 6 proximally causes clip guard 700 to disengage from catheter hub 300.

The thus removed clip guard 700 and needle housing 600 are shown in Figs. 14a-14c. Figs. 14c-14d show the positioning of needle tip protector 1110 inside chamber 712 of cylindrical portion 702 of clip guard 700. Moreover, shields 704a and 704b, which extend beyond the distal end 706 of cylindrical portion 702, together with upper cover 728 and lower cover 730 which extend beyond distal end 706 effectively surround needle tip protector 1110 to substantially isolate it from the environment. Fig. 14e is a semi-transparent side view of clip guard 700 showing needle tip protector 1100 being well protected by clip guard 700 after needle 6 has been withdrawn from catheter 4. Figs. 14a-14e show the safety catheter device in the locked safe position.

With reference to Figs. 7a-7f, 9a-9g and 13a-13d, the locking, or permanent retention of safety clip 1110 and clip cup 900 in chamber 712 of clip guard 700, is described herein. As particularly shown in Fig. 13c, in the ready to use position, the juncture where distal portion 902 and proximal portion 908 merge has a curved surface that is in contact with finger hook 720 of beam 722. When needle 6 is withdrawn in the proximal direction, after needle feature 6c abuts base 1102 of safety clip 1100, since base 910 of clip cup 900 is fixedly attached to base 1102, clip cup 900 is pulled in the proximal direction as finger hook 720 continues to be in contact with the outer surface of clip cup 900 due to the flexibility of cantilever beam 722. Once distal end 906 of clip cup 900 moves past finger hook 720, no longer being biased, finger hook 720 returns to its unbiased position such that its hook surface 720a comes into contact with and catches distal end 906 of clip cup 900 to prevent clip cup 900, and safety clip 1100 fixedly attached thereto, to move in the distal direction so as to reemerge from clip guard 700. As a result, safety clip 1100 and clip cup 900 are non-removably housed within clip guard 700, with the distal tip portion of needle 6 in turn housed in safety clip 1100.

With the safety clip 1110 enclosing the distal tip 6a of the needle and the clip guard 700 in turn protecting the safety clip removed from the catheter hub 300, a brief discussion is given herein of the coupling of the catheter hub with an external connector such as a luer for connecting the catheter hub to an external device such as a syringe of a pump with reference to Figs 15a-15c. Detailed discussion of the coupling of the catheter hub with a resealable seal member or valve such as that of the instant invention catheter assembly may be gleaned from the aforenoted incorporated by reference '104 US patent and '439 co-pending US application.

Fig. 15a shows the catheter hub 300 in alignment with a connector 150 such as a luer taper connector or a luer lock connector (conventionally referred to as luer taper and luer lock). Fig. 15b is a cross-sectional view of both catheter hub 300 and connector 150. As shown, connector 150 has a tapered nose portion 152 having a distal end 154. Catheter hub 300, as discussed above, has non-removably and fittingly provided at its interior cavity seal member 400 and actuator 500. Distal end 502 of actuator 500 is fixedly held at distal end portion 306 of catheter hub 300 along with catheter 4. As shown, the frusto-conical proximal portion 506 of actuator 500 is positioned in the bore defined by distal portion 404 that has the corrugated or accordion shaped configuration 406. Proximal end portion 506a of actuator 500, as shown in Fig. 15b, is in a sealing fit with inwardly protruding circumferential seal 422 at seal member 400. Proximal end 508 of actuator 500 is shown in Fig. 15b to be positioned distal of membrane 418 of seal member 400 after the needle has been removed from catheter hub 300, but before connector 150 is coupled to catheter hub 300. As described in the aforenoted '104 US patent and '439 co-pending US application, membrane 418 may have at least one resealable slit that facilitates its opening when membrane 418 is biased against the proximal open end 508 of actuator 500.

As shown in Fig. 15c, connector 150 and catheter hub 300 are coupled to each other, with tapered nose 152 inserted into the interior cavity of catheter hub 300 and its outer wall in slidable contact taper fit with the inner wall of the proximal portion of catheter hub 300. Distal end 154 of nose 152 comes into contact with the proximal end 416 of seal member 400 so that a distal force from connector, more precisely from the device that has connector 150, is imparted to seal member 400. Given that accordion shaped distal portion 406 is compressible under pressure, seal member 400 is shifted axially in the distal direction relative to catheter hub 300. As a result, membrane 418 is biased against the opened proximal end 508 of actuator 500. In Fig. 15c, assuming that connector is fully coupled to catheter hub 300, membrane 418, assisted by it slits, is opened by proximal end 508 of actuator such that opened proximal end 508 extends proximally past membrane 418 to establish a through fluid passage 158 between the catheter (Figs.2a-2b) extending from distal portion 306 of catheter hub 300 and the fluid container, for example a syringe or a fluid pump, to which connector 150 is a part of. Circumferential seal 422 prevents fluid from leaking around the frusto-conical portion 506 of actuator 500. When connector 150 is decoupled or disconnected from catheter hub 300, as per shown in Fig. 15b, due to the elastic characteristics of seal member 400 and the spring like characteristics of accordion distal portion 406, seal member 400, and particularly its proximal portion 402, is returned to its natural position by shifting axially in the proximal direction relative to catheter hub 300. No longer biased, membrane 418 is positioned proximal of opened proximal end 508 of actuator 500 and reseals to close off the fluid path 156 as shown in Fig. 15b.

A variant of the needle tip protector or safety clip shown in Figs. 11a-11e is shown in Fig. 16a. The illustrated safety clip 1100 is used in the safety catheter device shown in Figs. 16b-16f. Components that are the same as the embodiment shown in Figs. 13a-13d are labeled the same. The safety clip in Figs. 11a-11e and 16 is different from the safety clip described earlier in that there are two side shanks, prongs or tangs 1118 and 1120 that extend substantially orthogonally from base 1102 at an angle away from the longitudinal axis 1122 of the safety clip 1100. As shown in Figs. 16b-16d, when safety clip 1100 is partially located within the bore of the proximal portion 402 in catheter hub 300, due to the biasing of barriers 1108 and 1110 by needle 6, bosses 1112 and 1114 at arms 1104 and 1106, respectively, of safety clip 1100 are in pressing engagement with wall 410 of seal member 400 to hold the safety clip and clip guard 700 to catheter hub 300. When needle 6 is withdrawn from catheter hub 300 with its distal tip 6a housed inside safety clip 1100, since barriers 1108 and 1110 are no longer biased by needle 6, arms 1104 and 1106 would return to their natural position so that bosses 1112 and 1114 are released from wall 410 of seal member 400, as per shown in Fig. 16d.

With the needle feature 6c in contact with base 1102, safety clip 1100 is pulled in the proximal direction so that the proximal portion of safety clip 1100 which includes tangs 1118 and 1120 is positioned within the enlarged portion of chamber 712 of clip guard 700. For the embodiment shown in Figs. 16a-16f, chamber 712 is configured to have a circumferential shoulder 724 at the end of a reverse frusto-conical entrance 732 that allows tangs 1118 and 1120 to pass through in the proximal direction, as the tangs can be compressed when moving in the proximal direction. Once within the enlarged cavity of chamber 712 of clip guard 700, tangs 1118 and 1120 decompress and return to their outwardly angled natural position so that distal movement of the safety clip would cause the distal end edges 1118a and 1120a of tangs 1118 and 1120, respectively, to abut against shoulder 724 to prevent further distal movement of safety clip 1110 so as to prevent safety clip 1100 from reemerging from chamber 712. Since the contaminated distal tip of the needle is enclosed within safety clip 1100 and safety clip 1100 is prevented from moving distally by barriers 1108 and 1110, and further safety clip 1110 is non-movably housed in chamber 712 of clip guard 700, the distal tip of the needle is prevented from being exposed to the environment from the time the device is shipped to its use and subsequent disposal.

Figs. 17b-17e show the use of safety clip 1200 of Fig. 17a, which is a variation of the Figs. 12a-12e safety clip, in the safety catheter device. The part of safety clip 1200 that is different from safety clip 1100 is the box like structure 1230 at the proximal portion of safety clip 1200. Note that in Fig. 12f, the supports 1232a and 1232b are not shown, as the supports may not be needed if the box structure is made sufficiently rigid. Being box like, structure 1230 provides less free play and more readily guides safety clip 1200 into chamber 712, as its side walls 1232 and 1234 are dimensioned to fittingly slidable along side walls 712a and 712b of chamber 712, as shown in Fig. 17a and the b - - b view in Fig. 17b. As shown in Fig. 17d, shoulder 724, formed at the junction of the reverse frusto-conical opening 732 and chamber 712, acts as an obstruction to prevent safety clip 1200 from moving in the distal direction out of clip guard 700 once box structure 1230 has entered into chamber 712, since the respective distal edges of angled tangs 1218 and 1220 would abut against shoulder 724 as tangs 1218 and 1220 have returned to their natural outwardly angled position as previously described when safety clip 1200 enters chamber 712. Thus, between shoulder 724 and back wall or proximal end base 708, safety clip 1210 is non-removably housed in clip guard 700. Note that shoulder 724 may not be one continuous circumferential formation, as side walls 712a and 712b of chamber 712 are formed in parallel to each other to guide surfaces 1232 and 1234, respectively, of box structure 1230 into chamber 712. Once clip guard 700 is removed, decoupled or disconnected from catheter hub 300 as per shown in Fig. 17f, clip guard 700 and safety clip 1200 enclosed therein may be disposed of along with the needle housing 600 tethered to clip guard 700 by needle 6.

An alternative embodiment of the clip cup of the present invention is shown in Figs. 18a-18c. As shown, clip cup 1800 is a substantially cylindrical housing that has a distal portion 1802 having a distal opening 1806 and a proximal portion 1804. Distal portion 1802 is integrally joined to proximal portion 1804 by a shoulder 1808 that rises from distal portion 1802 to proximal portion 1804. The internal diameter of proximal portion 1804 is greater than that of distal portion 1802, which has the same diameter as distal opening 1806. Extending from proximal end base or wall 1810 of proximal portion 1804 is a shaft 1812 that has a proximal end flange or plate 1814. A bore 1816 connects a proximal end opening 1818 at shaft 1812 to the interior cavity or chamber 1820 of clip cup 1800 through an opening at the proximal end wall 1810. Bore 1816 has a cross section that enables a needle cannula to pass through but prevents the feature of the needle from passing.

Clip cup 1800 is part of the safety catheter device shown in Figs. 19a-19d. The components in the safety catheter device of the Figs. 19a-d embodiment that are the same as those described earlier are labeled the same. In addition to clip cup 1800, actuator 500 as shown in Figs. 5a-5e is also part of the safety catheter device shown in Figs. 19a-19d. With reference to Figs. 19b-19d, which are enlarged views focusing on the interaction between clip guard 700, safety clip 1100 and clip cup 1800, the safety catheter device is shown in a ready to use position or state. In particular, safety clip 1100 is shown to have its base 1102 secured to proximal end wall or base 1810 of clip cup 1800 in the manner as described earlier in the Figs. 13a-13d embodiment. Thus, safety clip 1100 and clip 1800 move in unison. In the ready to use position, shoulder 1808 of clip cup 1800 is in contact with the internal shoulder 723 (Fig. 7b) in chamber 712 of clip guard 700. This interaction of shoulder 1808 of clip cup 1800 with the internal shoulder 723 of clip guard 700 assists in stabilizing clip cup 1800 in clip guard 700. This is in addition to the stabilization provided by the distal end portion of clip cup 1800 being positioned between the proximal end portion of seal member 400 and the inner wall at the proximal end portion of catheter hub 300. As best shown in Figs. 19b and 19d, the chamfer edge of the proximal end wall 1810 of clip cup 1800 is in contact with the angled surface 720b of finger hook 720 to provide additional stability for clip cup 1800. In the ready to use position shown in Figs. 18a-18d, safety clip 1100, as needle 6 is passing therethrough, is fixedly enclosed in seal member 400 and clip cup1800. Together with safety clip 1100 housed therein, clip cup 1800 is encircled and isolated from the environment by clip guard 700.

Figs. 20a-20c are semi-transparent views showing the safety catheter device in a ready to use position, but with needle 6 being withdrawn in a proximal direction. As shown, as the clinician pulls the needle in the proximal direction relative to catheter hub 300, needle housing 600 begins to separate from the proximal end portion of clip guard 700, albeit the relationship between clip cup 1800 and clip guard 700 remains the same as before since safety clip 1100 continues to be biased by the being withdrawn needle 6.

Figs. 21a-21e show the safety catheter device being in the safe state of safe position whereby the clip guard 700 has separated from catheter hub 300. As shown, clip cup 1800 and safety clip 1100 attached thereto are housed within clip guard 700 where the distal tip 6a of needle 6 is enclosed by safety clip 1100 and prevented by barriers 1110 and 1108 from being further exposed in the distal direction. With reference to Figs. 21b and 21c, the combined proximal end walls or bases 1202 and 1810 of safety clip 1100 and clip cup 1800, respectively, is shown having been pulled by needle 6 (with needle feature 6c not shown) in the proximal direction relative to clip guard 700, such that the proximal end plate 1814 of clip cup 1800 is positioned between the proximal end wall 708 and catch stop 716 of clip guard 700. This results from the pulling of the needle in the proximal direction so that proximal end plate 1814 first makes contact with distal surface 716b of catch stop 716, then passes stop 716 and finally stopped by proximal end wall 708. Thus positioned, proximal end plate 1814 is trapped since it is prevented by the proximal surface 716a of catch stop 716 from moving in the distal direction. Clip cup 1800 remains stable within clip guard 700 due to the finger tip of finger hook 720 being in biasing contact with the outer surface of the distal portion 1802 of clip cup 1800. The proximal surface 720a of catch hook 720 would also prevent distal movement of clip cup 1800 in the distal direction when shoulder 1808 of clip cup 1800 comes into contact therewith. Thus, clip cup 1800, and safety clip 110 attached thereto, are non-removably housed within clip guard 700 in the safe position. Clip guard 700 and the needle housing 600 attached thereto, by way of the shaft of needle 6, may then be safely disposed of.

Fig. 21d is a semi-transparent view showing an opposite perspective view of the clip cup and the barriers of the safety clip inside clip guard 700. Fig. 21e is a solid view of Fig. 21d which in particular shows the irregularly shaped opening into cylindrical portion 702 that sandwiches extension 734 of catch stop 716. As described above, the opening may be necessary due to molding of clip guard 700.

It is the intension of the inventors that all matter described throughout this specification and shown in the accompanying drawings be interpreted as illustrative only and not in a limiting sense. For example, even though the distal portion of safety clip 1100 including the barriers 1208 and 1210 is shown to be inside the bore defined by the proximal portion of seal member 400, it should be appreciated that the bossed of the barriers may in practice be in engagement with an internal counter groove or shoulder at the inner wall of the catheter hub 300 when in the ready to use position. Thus, the safety clip of the present invention may co-operatively coact with the catheter hub without having to interact with the seal member.

## Claims

1. A safety catheter device comprising:
a catheter hub (300) having a body including an internal cavity (302) extending between a distal end (306) and an opened proximal end (310);
a seal member (400) having a substantially cylindrical wall (410) defining a bore (411) non-removably positioned in the internal cavity (302) of the catheter hub, a membrane (418) integral of the seal member partitioning the seal member into a hollow distal portion (404) and a hollow proximal portion (402) opened to the proximal end (310) of the catheter hub (300);
a catheter (4) having a distal end (4a) extending from the distal end (306) of the catheter hub (300) to establish a through passageway between the distal end of the catheter and the internal cavity (302) distal of the membrane (418);
a needle (6) having a proximal end attached to a needle hub (602) and a sharp distal tip (6a) adapted to extend through the catheter (4) until the distal tip of the needle extends beyond the distal end (4a) of the catheter;
a needle tip protector (1100, 1200) slidably positioned along the needle (6) having at least one barrier (1108. 1110) and opposing bosses (1112, 1114) being biased away from each other so as to be in fixed engagement with the wall (410) at the hollow proximal portion (402) of the seal member (400) when the distal tip (6a) of the needle is distal of the barrier (1108, 1110);
a guard (700) in coupled relationship with a proximal portion of the catheter hub (300) that includes the catheter hub proximal end (310) when the needle tip protector (1100, 1200) is in fixed engagement with the seal member (400), the guard having at least one extension (704a, 704b) that overlaps the proximal portion of the catheter hub (300) when the safety catheter device is in a ready to use position.

2. The safety catheter device of claim 1, wherein the guard (700) includes a chamber (712) configured to house the needle tip protector (1100, 1200), wherein when the distal tip (6a) of the needle (6) is positioned proximal the barrier (1108, 1110) such that the opposing bosses (1112, 1114) are no longer biased against the wall (410) of the seal member (400), further movement of the needle (6) in the proximal direction relative to the catheter hub (300) causes the needle tip protector (1100, 1200) to be housed in the chamber (712) and the guard (700) to decouple from the proximal portion of the catheter hub (300).

3. The safety catheter device of claim 2, wherein the guard (700) includes a shoulder (723) connecting the chamber (712) and the extension (704a, 704b) of the guard configured to abut the opened proximal end (310) of the catheter hub (300) when the safety catheter device is in the ready to use position.

4. The safety catheter device of claim 2, wherein the needle hub (602) is in a needle housing (600) having an opened distal receptacle (618) for receiving at least a proximal portion of the chamber (712) of the guard (700) when the safety catheter device is in the ready to use position.

5. The safety catheter device of any of the preceding claims, further comprising:
an actuator (500) positioned in the internal cavity (302) of the catheter hub (300) having a proximal end (508) extending proximally from the distal end (306) of the catheter hub (300), the proximal end (508) of the actuator positioned distal from the membrane (418) when the safety catheter device is in a ready to use position.

6. The safety catheter device of any of the preceding claims, wherein the distal portion (404) of the seal member (400) has an accordion shape and is compressible when the proximal end (416) of the seal member (400) is impacted by a distal force, the distal portion (404) of the seal member returning to its original shape when the distal force is removed.

7. The safety catheter device of claim 6, wherein the membrane (418) meets and is opened by the proximal end (508) of the actuator (500) when the distal force pushes the seal member (400) in the distal direction to move the membrane (418) into the proximal end of the actuator.

8. The safety catheter device of any of the preceding claims, further comprising:
a cup (900) for enclosing at least a portion of the needle tip protector (1100, 1200) having a proximal base (910) including an orifice (914) to enable the needle (6) to pass therethrough, the cup having a distal opening (904) for accepting the needle tip protector (1100, 1200) into the cup, the proximal base (910) of the cup fixedly attached to the base (1102) of the needle tip protector (1100) so that the cup (900) and the needle tip protector (1100) are movable in unison, the proximal base (910) of the cup (900) movably positioned into abutment with a proximal end of a chamber (712) of the guard (700) when the safety catheter device is in a safe position.

9. The safety catheter device of claim 8, wherein the cup (900) comprises a cylinder defined by a circumferential wall extending distally from its base (910), the distal end (906) of the wall held between a proximal end portion (416) of the seal member (400) and the interior cavity (302) of the catheter hub (300) when the safety catheter device is in the ready to use position.

10. The safety catheter device of claim 8, wherein the chamber (712) of the guard (700) has at least one stop (716) that biases against the distal end (906) of the tip protector cup (900) to prevent distal movement of the needle tip protector (1100) and cup (900) when the cup is fully retracted into the chamber.

11. The safety catheter device of claim 1 or claim 8, wherein the needle tip protector (1100) comprises a base (1102) having an opening (1116) for the needle (6) to pass through and two elongate arms (1104, 1106) each having one of the opposing bosses (1112, 1114) extending distally from the base, the arms biased by the needle such that the opposing bosses (1104, 1106) are pressed into the fixed engagement with the seal member (400) when the safety catheter device is in the ready to use position; wherein when the distal tip (6a) of the needle (6) is moved proximal the barrier (1108, 1110) of the tip protector, the arms (1104, 1106) being no longer biased by the needle would return to their natural position such that the opposing bosses (1104, 1106) are no longer fixedly engaged to the seal member.

12. The safety catheter device of claim 4, wherein the needle housing (600) comprises an elongated housing including a flash blood compartment (614) to receive fluid from the proximal end of the needle (6) attached to the needle hub (602), the flash blood compartment having a proximal end (610) closed by a flash plug (800).

13. A method of making a safety catheter device comprising the steps of:
providing a catheter hub (300) having a body including an internal cavity (302) extending between a distal end (306) and an opened proximal end (310);
non-removably positioning a seal member (400) having a substantially cylindrical wall (410) defining a bore (411) in the internal cavity (302) of the catheter hub (300), a membrane (418) integral of the seal member partitioning the seal member into a hollow distal portion (404) and a hollow proximal portion (402) opened to the proximal end (310) of the catheter hub (300);
connecting a catheter (4) having a distal end (4a) to the distal end (306) of the catheter hub (300) to establish a through passageway between the distal end of the catheter and the internal cavity (302) distal of the membrane (418);
attaching a proximal end of a needle (6) to a needle hub (602), the needle having a sharp distal tip (6a) adapted to extend through the catheter (4) until the distal tip (6a) of the needle extends beyond the distal end (4a) of the catheter;
slidably positioning a needle tip protector (1100, 1200) along the needle (6), the needle tip protector having at least one barrier (1108, 1110) and opposing bosses (1112, 1114);
positioning the distal tip (6a) of the needle distally of the barrier (1108, 1110) to bias the opposing bosses (1112, 1114) to be in fixed engagement with the wall (410) at the hollow proximal portion (402) of the seal member (400); and
coupling a guard (700) having at least one extension (704a, 704b) to a proximal portion of the catheter hub (300) when the needle tip protector (1100, 1200) is in fixed engagement with the seal member (400) so that the extension overlaps the proximal portion when the safety catheter device is in a ready to use position.

14. The method of claim 13, further comprising the step of:
providing a chamber (712) in the guard (700) configured to house the needle tip protector (1100, 1200) when the distal tip (6a) of the needle (6) is positioned proximal the barrier (1108, 1110) such that the opposing bosses (1112, 1114) are not biased against the wall (410) of the seal member (400) and further movement of the needle (6) in the proximal direction relative to the catheter hub (300) causes the needle tip protector (1100, 1200) to be housed in the chamber (712) and the guard (700) to decouple from the proximal portion of the catheter hub (300).

15. The method of claim 13 or claim 14, further comprising the steps of:
providing a cup (900) having a proximal base (910) including an orifice (914) to enable the needle (6) to pass therethrough for enclosing at least a portion of the needle tip protector (1100), the cup (900) having a distal opening (904) and a circumferential wall extending distally from the base (910) adapted to accept the needle tip protector (1100, 1200) into the cup;
fixedly attaching the proximal base (910) of the cup (900) to the base (1102) of the needle tip protector (1100) so that the cup (900) and the needle tip protector (1100) are movable in unison;
fittingly holding the distal end (906) of the wall between a proximal end portion (416) of the seal member (400) and the interior cavity (302) of the catheter hub (300) when the safety catheter device is in the ready to use position.

## Patentansprüche

1. Sicherheitskathetervorrichtung, umfassend:
eine Katheternabe (300), die einen Körper aufweist, der einen inneren Hohlraum (302) einschließt, der sich zwischen einem distalen Ende (306) und einem geöffneten proximalen Ende (310) erstreckt;
ein Dichtungselement (400), das eine im Wesentlichen zylindrische Wand (410) aufweist, die eine Bohrung (411) definiert, und nicht entfernbar in dem inneren Hohlraum (302) der Katheternabe positioniert ist, wobei eine mit dem Dichtungselement einstückige Membran (418) das Dichtungselement in einen hohlen distalen Abschnitt (404) und einen hohlen proximalen Abschnitt (402), der zu dem proximalen Ende (310) der Katheternabe (300) geöffnet ist, unterteilt;
einen Katheter (4), der ein distales Ende (4a) aufweist und sich von dem distalen Ende (306) der Katheternabe (300) erstreckt, um einen Durchgang zwischen dem distalen Ende des Katheters und dem inneren Hohlraum (302) distal der Membran (418) zu schaffen;
eine Nadel (6), die ein proximales Ende, das an einer Nadelnabe (602) angebracht ist, und eine scharfe distale Spitze (6a) aufweist, die dazu ausgelegt ist, sich durch den Katheter (4) zu erstrecken, bis sich die distale Spitze der Nadel über das distale Ende (4a) des Katheters hinaus erstreckt;
einen Nadelspitzenschutz (1100, 1200), der verschiebbar entlang der Nadel (6) positioniert ist und mindestens eine Barriere (1108. 1110) und gegenüberliegende Vorsprünge (1112, 1114) aufweist, die voneinander weg vorgespannt sind, damit sie sich in festem Eingriff mit der Wand (410) an dem hohlen proximalen Abschnitt (402) des Dichtungselements (400) befinden, wenn sich die distale Spitze (6a) der Nadel distal der Barriere (1108, 1110) befindet;
eine Schutzeinrichtung (700) in gekoppelter Beziehung mit einem proximalen Abschnitt der Katheternabe (300), der das proximale Ende (310) der Katheternabe einschließt, wenn sich der Nadelspitzenschutz (1100, 1200) in festem Eingriff mit dem Dichtungselement (400) befindet, wobei die Schutzeinrichtung mindestens eine Verlängerung (704a, 704b) aufweist, die den proximalen Abschnitt der Katheternabe (300) überlappt, wenn sich die Sicherheitskathetervorrichtung in einer verwendungsbereiten Position befindet.

2. Sicherheitskathetervorrichtung nach Anspruch 1, wobei die Schutzeinrichtung (700) eine Kammer (712) einschließt, die dazu konfiguriert ist, den Nadelspitzenschutz (1100, 1200) unterzubringen, wobei, wenn die distale Spitze (6a) der Nadel (6) derart proximal der Barriere (1108, 1110) positioniert ist, dass die gegenüberliegenden Vorsprünge (1112, 1114) nicht mehr gegen die Wand (410) des Dichtungselements (400) vorgespannt sind, eine weitere Bewegung der Nadel (6) in der proximalen Richtung relativ zu der Katheternabe (300) bewirkt, dass der Nadelspitzenschutz (1100, 1200) in der Kammer (712) untergebracht ist und die Schutzeinrichtung (700) sich von dem proximalen Abschnitt der Katheternabe (300) entkoppelt.

3. Sicherheitskathetervorrichtung nach Anspruch 2, wobei die Schutzeinrichtung (700) eine die Kammer (712) und die Verlängerung (704a, 704b) der Schutzeinrichtung verbindende Schulter (723) einschließt, die dazu konfiguriert ist, an dem geöffneten proximalen Ende (310) der Katheternabe (300) anzuliegen, wenn sich die Sicherheitskathetervorrichtung in der verwendungsbereiten Position befindet.

4. Sicherheitskathetervorrichtung nach Anspruch 2, wobei sich die Nadelnabe (602) in einem Nadelgehäuse (600) befindet, das eine geöffnete distale Aufnahme (618) zum Aufnehmen mindestens eines proximalen Abschnitts der Kammer (712) der Schutzeinrichtung (700) aufweist, wenn sich die Sicherheitskathetervorrichtung in der verwendungsbereiten Position befindet.

5. Sicherheitskathetervorrichtung nach einem der vorhergehenden Ansprüche, ferner umfassend:
einen Aktor (500), der in dem inneren Hohlraum (302) der Katheternabe (300) positioniert ist und ein proximales Ende (508) aufweist, das sich proximal von dem distalen Ende (306) der Katheternabe (300) erstreckt, wobei das proximale Ende (508) des Aktors distal von der Membran (418) positioniert ist, wenn sich die Sicherheitskathetervorrichtung in einer verwendungsbereiten Position befindet.

6. Sicherheitskathetervorrichtung nach einem der vorhergehenden Ansprüche, wobei der distale Abschnitt (404) des Dichtungselements (400) eine Akkordeonform aufweist und komprimierbar ist, wenn das proximale Ende (416) des Dichtungselements (400) durch eine distale Kraft beaufschlagt wird, wobei der distale Abschnitt (404) des Dichtungselements in seine ursprüngliche Form zurückkehrt, wenn die distale Kraft entfernt wird.

7. Sicherheitskathetervorrichtung nach Anspruch 6, wobei die Membran (418) auf das proximale Ende (508) des Aktors (500) trifft und durch diesen geöffnet wird, wenn die distale Kraft das Dichtungselement (400) in die distale Richtung drückt, um die Membran (418) in das proximale Ende des Aktors zu bewegen.

8. Sicherheitskathetervorrichtung nach einem der vorhergehenden Ansprüche, ferner umfassend:
einen Becher (900) zum Umschließen mindestens eines Abschnitts des Nadelspitzenschutzes (1100, 1200), der eine proximale Basis (910) aufweist, die einen Durchlass (914) einschließt, um es der Nadel (6) zu ermöglichen, durch diesen zu verlaufen, wobei der Becher eine distale Öffnung (904) zum Akzeptieren des Nadelspitzenschutzes (1100, 1200) in dem Becher aufweist, wobei die proximale Basis (910) des Bechers fest an der Basis (1102) des Nadelspitzenschutzes (1100) angebracht ist, sodass der Becher (900) und der Nadelspitzenschutz (1100) gemeinsam bewegbar sind, wobei die proximale Basis (910) des Bechers (900) bewegbar in Anlage an einem proximalen Ende einer Kammer (712) der Schutzeinrichtung (700) positioniert wird, wenn sich die Sicherheitskathetervorrichtung in einer sicheren Position befindet.

9. Sicherheitskathetervorrichtung nach Anspruch 8, wobei der Becher (900) einen Zylinder umfasst, der durch eine umlaufende Wand definiert ist, die sich distal von seiner Basis (910) erstreckt, wobei das distale Ende (906) der Wand zwischen einem proximalen Endabschnitt (416) des Dichtungselements (400) und dem Innenhohlraum (302) der Katheternabe (300) gehalten wird, wenn sich die Sicherheitskathetervorrichtung in der verwendungsbereiten Position befindet.

10. Sicherheitskathetervorrichtung nach Anspruch 8, wobei die Kammer (712) der Schutzeinrichtung (700) mindestens einen Anschlag (716) aufweist, der gegen das distale Ende (906) des Spitzenschutzbechers (900) vorspannt, um eine distale Bewegung des Nadelspitzenschutzes (1100) und des Bechers (900) zu verhindern, wenn der Becher vollständig in die Kammer zurückgezogen ist.

11. Sicherheitskathetervorrichtung nach Anspruch 1 oder Anspruch 8, wobei der Nadelspitzenschutz (1100) eine Basis (1102), die eine Öffnung (1116) für einen Verlauf der Nadel (6) durch diese aufweist, und zwei längliche Arme (1104, 1106) umfasst, die jeweils einen der gegenüberliegenden Vorsprünge (1112, 1114) aufweisen, die sich distal von der Basis erstrecken, wobei die Arme durch die Nadel derart vorgespannt sind, dass die gegenüberliegenden Vorsprünge (1104, 1106) in den festen Eingriff mit dem Dichtungselement (400) gepresst werden, wenn sich die Sicherheitskathetervorrichtung in der verwendungsbereiten Position befindet; wobei, wenn die distale Spitze (6a) der Nadel (6) proximal der Barriere (1108, 1110) des Spitzenschutzes bewegt wird, die Arme (1104, 1106), die nicht mehr durch die Nadel vorgespannt sind, derart in ihre natürliche Position zurückkehren würden, dass die gegenüberliegenden Vorsprünge (1104, 1106) nicht mehr fest mit dem Dichtungselement in Eingriff stehen.

12. Sicherheitskathetervorrichtung nach Anspruch 4, wobei das Nadelgehäuse (600) ein längliches Gehäuse umfasst, das einen Flash-Blutraum (614) einschließt, um Fluid von dem proximalen Ende der Nadel (6) aufzunehmen, die an der Nadelnabe (602) angebracht ist, wobei der Flash-Blutraum ein proximales Ende (610) aufweist, das durch einen Flash-Stecker (800) geschlossen ist.

13. Verfahren zum Herstellen einer Sicherheitskathetervorrichtung, umfassend die folgenden Schritte:
Bereitstellen einer Katheternabe (300), die einen Körper aufweist, der einen inneren Hohlraum (302) einschließt, der sich zwischen einem distalen Ende (306) und einem geöffneten proximalen Ende (310) erstreckt;
nicht entfernbares Positionieren eines Dichtungselements (400), das eine im Wesentlichen zylindrische Wand (410) aufweist, die eine Bohrung (411) definiert, in dem inneren Hohlraum (302) der Katheternabe (300), wobei eine mit dem Dichtungselement einstückige Membran (418) das Dichtungselement in einen hohlen distalen Abschnitt (404) und einen hohlen proximalen Abschnitt (402), der zu dem proximalen Ende (310) der Katheternabe (300) geöffnet ist, unterteilt;
Verbinden eines Katheters (4), der ein distales Ende (4a) aufweist, mit dem distalen Ende (306) der Katheternabe (300), um einen Durchgang zwischen dem distalen Ende des Katheters und dem inneren Hohlraum (302) distal der Membran (418) zu schaffen;
Anbringen eines proximalen Endes einer Nadel (6) an einer Nadelnabe (602), wobei die Nadel eine scharfe distale Spitze (6a) aufweist, die dazu ausgelegt ist, sich durch den Katheter (4) zu erstrecken, bis sich die distale Spitze (6a) der Nadel über das distale Ende (4a) des Katheters hinaus erstreckt;
verschiebbares Positionieren eines Nadelspitzenschutzes (1100, 1200) entlang der Nadel (6), wobei der Nadelspitzenschutz mindestens eine Barriere (1108, 1110) und gegenüberliegende Vorsprünge (1112, 1114) aufweist;
Positionieren der distalen Spitze (6a) der Nadel distal der Barriere (1108, 1110), um die gegenüberliegenden Vorsprünge (1112, 1114) vorzuspannen, damit sie sich in festem Eingriff mit der Wand (410) an dem hohlen proximalen Abschnitt (402) des Dichtungselements (400) befinden; und
Koppeln einer Schutzeinrichtung (700), die mindestens eine Verlängerung (704a, 704b) aufweist, an einen proximalen Abschnitt der Katheternabe (300), wenn sich der Nadelspitzenschutz (1100, 1200) in festem Eingriff mit dem Dichtungselement (400) befindet, sodass die Verlängerung den proximalen Abschnitt überlappt, wenn sich die Sicherheitskathetervorrichtung in einer verwendungsbereiten Position befindet.

14. Verfahren nach Anspruch 13, ferner umfassend den folgenden Schritt:
Bereitstellen einer Kammer (712) in der Schutzeinrichtung (700), die dazu konfiguriert ist, den Nadelspitzenschutz (1100, 1200) unterzubringen, wenn die distale Spitze (6a) der Nadel (6) derart proximal der Barriere (1108, 1110) positioniert ist, dass die gegenüberliegenden Vorsprünge (1112, 1114) nicht gegen die Wand (410) des Dichtungselements (400) vorgespannt sind und eine weitere Bewegung der Nadel (6) in der proximalen Richtung relativ zu der Katheternabe (300) bewirkt, dass der Nadelspitzenschutz (1100, 1200) in der Kammer (712) untergebracht ist und die Schutzeinrichtung (700) sich von dem proximalen Abschnitt der Katheternabe (300) entkoppelt.

15. Verfahren nach Anspruch 13 oder Anspruch 14, ferner umfassend die folgenden Schritte:
Bereitstellen eines Bechers (900), der eine proximale Basis (910) aufweist, die einen Durchlass (914) einschließt, um es der Nadel (6) zu ermöglichen, durch diesen zu verlaufen, zum Umschließen mindestens eines Abschnitts des Nadelspitzenschutzes (1100), wobei der Becher (900) eine distale Öffnung (904) und eine umlaufende Wand aufweist, die sich distal von der Basis (910) erstreckt und dazu ausgelegt ist, den Nadelspitzenschutz (1100, 1200) in dem Becher zu akzeptieren;
festes Anbringen der proximalen Basis (910) des Bechers (900) an der Basis (1102) des Nadelspitzenschutzes (1100), sodass der Becher (900) und der Nadelspitzenschutz (1100) gemeinsam bewegbar sind;
passendes Halten des distalen Endes (906) der Wand zwischen einem proximalen Endabschnitt (416) des Dichtungselements (400) und dem Innenhohlraum (302) der Katheternabe (300), wenn sich die Sicherheitskathetervorrichtung in der verwendungsbereiten Position befindet.

## Revendications

1. Dispositif de cathéter de sécurité, comprenant :
une embase de cathéter (300) possédant un corps comprenant une cavité interne (302) s'étendant entre une extrémité distale (306) et une extrémité proximale ouverte (310) ;
un élément d'étanchéité (400) comportant une paroi sensiblement cylindrique (410) définissant un alésage (411) positionné de manière inamovible dans la cavité interne (302) de l'embase de cathéter, une membrane (418) faisant partie intégrante de l'élément d'étanchéité divisant l'élément d'étanchéité en une partie distale creuse (404) et une partie proximale creuse (402) ouverte vers l'extrémité proximale (310) de l'embase de cathéter (300) ;
un cathéter (4) possédant une extrémité distale (4a) s'étendant à partir de l'extrémité distale (306) de l'embase de cathéter (300) de manière à établir un passage traversant entre l'extrémité distale du cathéter et la cavité interne (302) distale par rapport à la membrane (418) ;
une aiguille (6) possédant une extrémité proximale fixée à une embase d'aiguille (602) et une pointe distale pointue (6a) conçue pour s'étendre à travers le cathéter (4) jusqu'à ce que la pointe distale de l'aiguille s'étende au-delà de l'extrémité distale (4a) du cathéter ;
un protecteur de pointe d'aiguille (1100, 1200) positionné de manière coulissante le long de l'aiguille (6) comportant au moins une barrière (1108, 1110) et des bossages opposés (1112, 1114) étant sollicités à l'écart l'un de l'autre de façon à être en prise fixe avec la paroi (410) au niveau de la partie proximale creuse (402) de l'élément d'étanchéité (400) lorsque la pointe distale (6a) de l'aiguille est distale par rapport à la barrière (1108, 1110) ;
une protection (700) en relation couplée avec une partie proximale de l'embase de cathéter (300) qui comprend l'extrémité proximale de l'embase de cathéter (310) lorsque le protecteur de pointe d'aiguille (1100, 1200) se trouve en prise fixe avec l'élément d'étanchéité (400), la protection comportant au moins une extension (704a, 704b) qui chevauche la partie proximale de l'embase de cathéter (300) lorsque le dispositif de cathéter de sécurité se trouve dans une position prête à l'emploi.

2. Dispositif de cathéter de sécurité selon la revendication 1, ladite protection (700) comprenant une chambre (712) conçue pour loger le protecteur de pointe d'aiguille (1100, 1200) ; lorsque la pointe distale (6a) de l'aiguille (6) est positionnée proximale par rapport à la barrière (1108, 1110) de sorte que les bossages opposés (1112, 1114) ne soient plus sollicités contre la paroi (410) de l'élément d'étanchéité (400), un déplacement supplémentaire de l'aiguille (6) suivant la direction proximale par rapport à l'embase de cathéter (300) amenant le protecteur de pointe d'aiguille (1100, 1200) à être logé dans la chambre (712) et la protection (700) à se découpler de la partie proximale de l'embase de cathéter (300).

3. Dispositif de cathéter de sécurité selon la revendication 2, ladite protection (700) comprenant un épaulement (723) reliant la chambre (712) et ladite extension (704a, 704b) de la protection étant conçue pour venir en butée contre l'extrémité proximale ouverte (310) de l'embase de cathéter (300) lorsque le dispositif de cathéter de sécurité se trouve en position prête à l'emploi.

4. Dispositif de cathéter de sécurité selon la revendication 2, ladite embase d'aiguille (602) se trouvant dans un logement d'aiguille (600) comportant un réceptacle distal ouvert (618) permettant de recevoir au moins une partie proximale de la chambre (712) de la protection (700) lorsque le dispositif de cathéter de sécurité se trouve dans la position prête à l'emploi.

5. Dispositif de cathéter de sécurité selon l'une quelconque des revendications précédentes, comprenant en outre :
un actionneur (500) positionné dans la cavité interne (302) de l'embase de cathéter (300) comportant une extrémité proximale (508) s'étendant de manière proximale à partir de l'extrémité distale (306) de l'embase de cathéter (300), l'extrémité proximale (508) de l'actionneur étant positionnée de manière distale par rapport à la membrane (418) lorsque le dispositif de cathéter de sécurité se trouve dans une position prête à l'emploi.

6. Dispositif de cathéter de sécurité selon l'une quelconque des revendications précédentes, ladite partie distale (404) de l'élément d'étanchéité (400) présentant une forme d'accordéon et étant compressible lorsque l'extrémité proximale (416) de l'élément d'étanchéité (400) est touchée par une force distale, ladite partie distale (404) de l'élément d'étanchéité revenant à sa forme originale lorsque la force distale est supprimée.

7. Dispositif de cathéter de sécurité selon la revendication 6, ladite membrane (418) se rejoignant et étant ouverte par l'extrémité proximale (508) de l'actionneur (500) lorsque la force distale pousse l'élément d'étanchéité (400) dans la direction distale de manière à déplacer la membrane (418) dans l'extrémité proximale de l'actionneur.

8. Dispositif de cathéter de sécurité selon l'une quelconque des revendications précédentes, comprenant en outre :
une coupelle (900) permettant d'entourer au moins une partie du protecteur de pointe d'aiguille (1100, 1200) comportant une base proximale (910) comprenant un orifice (914) destiné à permettre le passage de l'aiguille (6) à travers elle, la coupelle comportant une ouverture distale (904) destinée à recevoir le protecteur de pointe d'aiguille (1100, 1200) dans la coupelle, la base proximale (910) de la coupelle étant fixée à demeure à la base (1102) du protecteur de pointe d'aiguille (1100) de sorte que la coupelle (900) et le protecteur de pointe d'aiguille (1100) soient mobiles à l'unisson, la base proximale (910) de la coupelle (900) étant positionnée de manière mobile en butée contre une extrémité proximale d'une chambre (712) de la protection (700) lorsque le dispositif de cathéter de sécurité se trouve dans une position sécurisée.

9. Dispositif de cathéter de sécurité selon la revendication 8, ladite coupelle (900) comprenant un cylindre défini par une paroi circonférentielle s'étendant de manière distale à partir de sa base (910), ladite extrémité distale (906) de la paroi étant maintenue entre une partie d'extrémité proximale (416) de l'élément d'étanchéité (400) et la cavité interne (302) de l'embase de cathéter (300) lorsque le dispositif de cathéter de sécurité se trouve dans la position prête à l'emploi.

10. Dispositif de cathéter de sécurité selon la revendication 8, ladite chambre (712) de la protection (700) comportant au moins une butée (716) qui sollicite contre l'extrémité distale (906) de la coupelle de protection de pointe (900) de manière à empêcher un déplacement distal de la protection de pointe d'aiguille (1100) et de la coupelle (900) lorsque la coupelle est complètement rétractée dans la chambre.

11. Dispositif de cathéter de sécurité selon la revendication 1 ou la revendication 8, ledit protecteur de pointe d'aiguille (1100) comprenant une base (1102) comportant une ouverture (1116) pour le passage de l'aiguille (6) et deux bras allongés (1104, 1106) comportant chacun l'un des bossages opposés (1112, 1114) s'étendant de manière distale à partir de la base, lesdits bras étant sollicités par l'aiguille de sorte que les bossages opposés (1104, 1106) soient pressés en prise fixe avec l'élément d'étanchéité (400) lorsque le dispositif de cathéter de sécurité se trouve en position prête à l'emploi ; lorsque la pointe distale (6a) de l'aiguille (6) est déplacée proximale par rapport à la barrière (1108, 1110) du protecteur de pointe, lesdits bras (1104, 1106) n'étant plus sollicités par l'aiguille revenant à leur position naturelle de sorte que les bossages opposés (1104, 1106) ne soient plus en prise à demeure avec l'élément d'étanchéité.

12. Dispositif de cathéter de sécurité selon la revendication 4, ledit logement d'aiguille (600) comprenant un logement allongé comprenant un compartiment de sang de détente (614) destiné à recevoir un fluide provenant de l'extrémité proximale de l'aiguille (6) fixée à l'embase d'aiguille (602), ledit compartiment de sang de détente possédant une extrémité proximale (610) fermée par un bouchon de détente (800).

13. Procédé de fabrication d'un dispositif de cathéter de sécurité comprenant les étapes de :
fourniture d'une embase de cathéter (300) possédant un corps comprenant une cavité interne (302) s'étendant entre une extrémité distale (306) et une extrémité proximale ouverte (310) ;
positionnement inamovible d'un élément d'étanchéité (400) comportant une paroi sensiblement cylindrique (410) définissant un alésage (411) dans la cavité interne (302) de l'embase de cathéter (300), une membrane (418) faisant partie intégrante de l'élément d'étanchéité divisant l'élément d'étanchéité en une partie distale creuse (404) et une partie proximale creuse (402) ouverte vers l'extrémité proximale (310) de l'embase de cathéter (300) ;
raccordement d'un cathéter (4) possédant une extrémité distale (4a) à l'extrémité distale (306) de l'embase de cathéter (300) de manière à établir un passage traversant entre l'extrémité distale du cathéter et la cavité interne (302) distale de la membrane (418) ;
fixation d'une extrémité proximale d'une aiguille (6) à une embase d'aiguille (602), l'aiguille possédant une pointe distale pointue (6a) adaptée pour s'étendre à travers le cathéter (4) jusqu'à ce que la pointe distale (6a) de l'aiguille s'étende au-delà de l'extrémité distale (4a) du cathéter ;
positionnement de manière coulissante d'un protecteur de pointe d'aiguille (1100, 1200) le long de l'aiguille (6), le protecteur de pointe d'aiguille comportant au moins une barrière (1108, 1110) et des bossages opposés (1112, 1114) ;
positionnement de la pointe distale (6a) de l'aiguille de manière distale par rapport à la barrière (1108, 1110) de manière à solliciter les bossages opposés (1112, 1114) afin qu'ils soient en prise fixe avec la paroi (410) au niveau de la partie proximale creuse (402) de l'élément d'étanchéité (400) ; et
couplage d'une protection (700) comportant au moins une extension (704a, 704b) à une partie proximale de l'embase de cathéter (300) lorsque le protecteur de pointe d'aiguille (1100, 1200) se trouve en prise fixe avec l'élément d'étanchéité (400) de sorte que l'extension chevauche la partie proximale lorsque le dispositif de cathéter de sécurité se trouve dans une position prête à l'emploi.

14. Procédé selon la revendication 13, comprenant en outre l'étape suivante :
fourniture d'une chambre (712) dans la protection (700) conçue pour loger le protecteur de pointe d'aiguille (1100, 1200) lorsque la pointe distale (6a) de l'aiguille (6) est positionnée proximale par rapport à la barrière (1108, 1110) de sorte que les bossages opposés (1112, 1114) ne soient pas sollicités contre la paroi (410) de l'élément d'étanchéité (400) et qu'un déplacement supplémentaire de l'aiguille (6) suivant la direction proximale par rapport à l'embase de cathéter (300) amène le protecteur de pointe d'aiguille (1100, 1200) à être logé dans la chambre (712) et la protection (700) à se découpler de la partie proximale de l'embase de cathéter (300).

15. Procédé selon la revendication 13 ou la revendication 14, comprenant en outre les étapes de :
fourniture d'une coupelle (900) comportant une base proximale (910) comprenant un orifice (914) destiné à permettre le passage de l'aiguille (6) à travers elle de manière à entourer au moins une partie du protecteur de pointe d'aiguille (1100), la coupelle (900) comportant une ouverture distale (904) et une paroi circonférentielle s'étendant de manière distale à partir de la base (910) adaptée pour recevoir le protecteur de pointe d'aiguille (1100, 1200) dans la coupelle ;
fixation à demeure de la base proximale (910) de la coupelle (900) à la base (1102) du protecteur de pointe d'aiguille (1100) de sorte que la coupelle (900) et le protecteur de pointe d'aiguille (1100) soient mobiles à l'unisson ;
maintien de manière ajustée de l'extrémité distale (906) de la paroi entre une partie d'extrémité proximale (416) de l'élément d'étanchéité (400) et la cavité interne (302) de l'embase de cathéter (300) lorsque le dispositif de cathéter de sécurité se trouve dans la position prête à l'emploi.
